# EUROPEAN PATENT APPLICATION

(11) **EP 3 940 075 A1**
(43) Date of publication of application: **19.01.2022**
(21) Application number: 20186492.3
(22) Date of filing: 17.07.2020
(51) Int. Cl.: C12N 15/113, A61K 31/7088, A61P 35/00

(54) **INHIBITORS OF LINE1 AND USES THEREOF**

(71) Applicant: Istituto Nazionale Di Genetica Molecolare-INGM, 20122 Milano (IT)
(72) Inventor: Bodega, Beatrice, 20122 Milano (IT); Marasca, Federica, 20122 Milano (IT)
(74) Representative: Turri, Elisa

(57) **Abstract**

The present invention relates to a suppressor or inhibitor of (long interspersed element 1) LINE1 (L1) expression for medical use.

SEQUENCE LISTING

<110>  Istituto Nazionale di Genetica Molecolare

<120>  INHIBITORS OF LINE1 AND USES THEREOF

<130>  BE 142925

<160>  6

<170>  PatentIn version 3.5

<210>  1
<211>  7104
<212>  DNA
<213>  Homo sapiens



<220>
<221>  misc_feature
<222>  (2)..(307)
<223>  n is a, c, g, or t

<220>
<221>  misc_feature
<222>  (381)..(381)
<223>  n is a, c, g, or t

<220>
<221>  misc_feature
<222>  (386)..(386)
<223>  n is a, c, g, or t

<220>
<221>  misc_feature
<222>  (510)..(512)
<223>  n is a, c, g, or t

<220>
<221>  misc_feature
<222>  (568)..(568)
<223>  n is a, c, g, or t

<220>
<221>  misc_feature
<222>  (662)..(663)
<223>  n is a, c, g, or t

<220>
<221>  misc_feature
<222>  (800)..(800)
<223>  n is a, c, g, or t

<220>
<221>  misc_feature
<222>  (930)..(930)
<223>  n is a, c, g, or t

<220>
<221>  misc_feature
<222>  (1087)..(1087)
<223>  n is a, c, g, or t

<220>
<221>  misc_feature
<222>  (1154)..(1155)
<223>  n is a, c, g, or t

<220>
<221>  misc_feature
<222>  (1280)..(1280)
<223>  n is a, c, g, or t

<220>
<221>  misc_feature
<222>  (1342)..(1342)
<223>  n is a, c, g, or t

<220>
<221>  misc_feature
<222>  (1379)..(1387)
<223>  n is a, c, g, or t

<220>
<221>  misc_feature
<222>  (1630)..(1630)
<223>  n is a, c, g, or t

<220>
<221>  misc_feature
<222>  (2114)..(2116)
<223>  n is a, c, g, or t

<220>
<221>  misc_feature
<222>  (2145)..(2145)
<223>  n is a, c, g, or t

<220>
<221>  misc_feature
<222>  (2186)..(2187)
<223>  n is a, c, g, or t

<220>
<221>  misc_feature
<222>  (2195)..(2195)
<223>  n is a, c, g, or t

<220>
<221>  misc_feature
<222>  (2238)..(2238)
<223>  n is a, c, g, or t

<220>
<221>  misc_feature
<222>  (2467)..(2467)
<223>  n is a, c, g, or t

<220>
<221>  misc_feature
<222>  (2471)..(2471)
<223>  n is a, c, g, or t

<220>
<221>  misc_feature
<222>  (2669)..(2672)

<223>  n is a, c, g, or t

<220>
<221>  misc_feature
<222>  (2843)..(2844)
<223>  n is a, c, g, or t

<220>
<221>  misc_feature
<222>  (2868)..(2868)
<223>  n is a, c, g, or t

<220>
<221>  misc_feature
<222>  (2921)..(2921)
<223>  n is a, c, g, or t

<220>
<221>  misc_feature
<222>  (2966)..(2968)
<223>  n is a, c, g, or t

<220>
<221>  misc_feature
<222>  (3113)..(3113)
<223>  n is a, c, g, or t

<220>
<221>  misc_feature
<222>  (3166)..(3168)
<223>  n is a, c, g, or t

<220>
<221>  misc_feature
<222>  (3202)..(3204)
<223>  n is a, c, g, or t

<220>
<221>  misc_feature
<222>  (3358)..(3358)
<223>  n is a, c, g, or t

<220>
<221>  misc_feature
<222>  (3391)..(3391)
<223>  n is a, c, g, or t

<220>
<221>  misc_feature
<222>  (3484)..(3484)
<223>  n is a, c, g, or t

<220>
<221>  misc_feature
<222>  (3613)..(3615)
<223>  n is a, c, g, or t

<220>
<221>  misc_feature
<222>  (3639)..(3639)
<223>  n is a, c, g, or t

<220>

<221>  misc_feature
<222>  (3689)..(3689)
<223>  n is a, c, g, or t

<220>
<221>  misc_feature
<222>  (3723)..(3723)
<223>  n is a, c, g, or t

<220>
<221>  misc_feature
<222>  (3735)..(3737)
<223>  n is a, c, g, or t

<220>
<221>  misc_feature
<222>  (3793)..(3793)
<223>  n is a, c, g, or t

<220>
<221>  misc_feature
<222>  (3795)..(3797)
<223>  n is a, c, g, or t

<220>
<221>  misc_feature
<222>  (3854)..(3860)
<223>  n is a, c, g, or t

<220>
<221>  misc_feature
<222>  (3888)..(3888)
<223>  n is a, c, g, or t

<220>
<221>  misc_feature
<222>  (3929)..(3929)
<223>  n is a, c, g, or t

<220>
<221>  misc_feature
<222>  (3954)..(3958)
<223>  n is a, c, g, or t

<220>
<221>  misc_feature
<222>  (3968)..(3968)
<223>  n is a, c, g, or t

<220>
<221>  misc_feature
<222>  (3990)..(3994)
<223>  n is a, c, g, or t

<220>
<221>  misc_feature
<222>  (3996)..(4001)
<223>  n is a, c, g, or t

<220>
<221>  misc_feature
<222>  (4084)..(4084)
<223>  n is a, c, g, or t

<220>
<221>  misc_feature
<222>  (4118)..(4119)
<223>  n is a, c, g, or t

<220>
<221>  misc_feature
<222>  (4154)..(4154)
<223>  n is a, c, g, or t

<220>
<221>  misc_feature
<222>  (4185)..(4186)
<223>  n is a, c, g, or t

<220>
<221>  misc_feature
<222>  (4205)..(4205)
<223>  n is a, c, g, or t

<220>
<221>  misc_feature
<222>  (4254)..(4255)
<223>  n is a, c, g, or t

<220>
<221>  misc_feature
<222>  (4351)..(4351)
<223>  n is a, c, g, or t

<220>
<221>  misc_feature
<222>  (4364)..(4364)
<223>  n is a, c, g, or t

<220>
<221>  misc_feature
<222>  (4377)..(4377)
<223>  n is a, c, g, or t

<220>
<221>  misc_feature
<222>  (4407)..(4410)
<223>  n is a, c, g, or t

<220>
<221>  misc_feature
<222>  (4423)..(4424)
<223>  n is a, c, g, or t

<220>
<221>  misc_feature
<222>  (4431)..(4432)
<223>  n is a, c, g, or t

<220>
<221>  misc_feature
<222>  (4533)..(4533)
<223>  n is a, c, g, or t

<220>
<221>  misc_feature

<222>  (4564)..(4564)
<223>  n is a, c, g, or t

<220>
<221>  misc_feature
<222>  (4612)..(4612)
<223>  n is a, c, g, or t

<220>
<221>  misc_feature
<222>  (4678)..(4679)
<223>  n is a, c, g, or t

<220>
<221>  misc_feature
<222>  (4685)..(4688)
<223>  n is a, c, g, or t

<220>
<221>  misc_feature
<222>  (4705)..(4706)
<223>  n is a, c, g, or t

<220>
<221>  misc_feature
<222>  (4880)..(4880)
<223>  n is a, c, g, or t

<220>
<221>  misc_feature
<222>  (4941)..(4944)
<223>  n is a, c, g, or t

<220>
<221>  misc_feature
<222>  (4989)..(4989)
<223>  n is a, c, g, or t

<220>
<221>  misc_feature
<222>  (5057)..(5058)
<223>  n is a, c, g, or t

<220>
<221>  misc_feature
<222>  (5094)..(5094)
<223>  n is a, c, g, or t

<220>
<221>  misc_feature
<222>  (5183)..(5183)
<223>  n is a, c, g, or t

<220>
<221>  misc_feature
<222>  (5243)..(5243)
<223>  n is a, c, g, or t

<220>
<221>  misc_feature
<222>  (5313)..(5316)
<223>  n is a, c, g, or t

<220>
<221>  misc_feature
<222>  (5320)..(5323)
<223>  n is a, c, g, or t

<220>
<221>  misc_feature
<222>  (5366)..(5369)
<223>  n is a, c, g, or t

<220>
<221>  misc_feature
<222>  (5402)..(5403)
<223>  n is a, c, g, or t

<220>
<221>  misc_feature
<222>  (5433)..(5437)
<223>  n is a, c, g, or t

<220>
<221>  misc_feature
<222>  (5485)..(5487)
<223>  n is a, c, g, or t

<220>
<221>  misc_feature
<222>  (5544)..(5545)
<223>  n is a, c, g, or t

<220>
<221>  misc_feature
<222>  (5594)..(5594)
<223>  n is a, c, g, or t

<220>
<221>  misc_feature
<222>  (5647)..(5649)
<223>  n is a, c, g, or t

<220>
<221>  misc_feature
<222>  (5728)..(5728)
<223>  n is a, c, g, or t

<220>
<221>  misc_feature
<222>  (5813)..(5814)
<223>  n is a, c, g, or t

<220>
<221>  misc_feature
<222>  (5828)..(5828)
<223>  n is a, c, g, or t

<220>
<221>  misc_feature
<222>  (5837)..(5837)
<223>  n is a, c, g, or t

<220>
<221>  misc_feature
<222>  (5871)..(5871)

<223>  n is a, c, g, or t

<220>
<221>  misc_feature
<222>  (5942)..(5943)
<223>  n is a, c, g, or t

<220>
<221>  misc_feature
<222>  (5962)..(5962)
<223>  n is a, c, g, or t

<220>
<221>  misc_feature
<222>  (6000)..(6003)
<223>  n is a, c, g, or t

<220>
<221>  misc_feature
<222>  (6015)..(6015)
<223>  n is a, c, g, or t

<220>
<221>  misc_feature
<222>  (6038)..(6039)
<223>  n is a, c, g, or t

<220>
<221>  misc_feature
<222>  (6062)..(6063)
<223>  n is a, c, g, or t

<220>
<221>  misc_feature
<222>  (6083)..(6086)
<223>  n is a, c, g, or t

<220>
<221>  misc_feature
<222>  (6090)..(6095)
<223>  n is a, c, g, or t

<220>
<221>  misc_feature
<222>  (6123)..(6125)
<223>  n is a, c, g, or t

<220>
<221>  misc_feature
<222>  (6136)..(6136)
<223>  n is a, c, g, or t

<220>
<221>  misc_feature
<222>  (6182)..(6183)
<223>  n is a, c, g, or t

<220>
<221>  misc_feature
<222>  (6195)..(6199)
<223>  n is a, c, g, or t

<220>

<221>  misc_feature
<222>  (6251)..(6255)
<223>  n is a, c, g, or t

<220>
<221>  misc_feature
<222>  (6259)..(6260)
<223>  n is a, c, g, or t

<220>
<221>  misc_feature
<222>  (6309)..(6311)
<223>  n is a, c, g, or t

<220>
<221>  misc_feature
<222>  (6337)..(6338)
<223>  n is a, c, g, or t

<220>
<221>  misc_feature
<222>  (6344)..(6344)
<223>  n is a, c, g, or t

<220>
<221>  misc_feature
<222>  (6386)..(6394)
<223>  n is a, c, g, or t

<220>
<221>  misc_feature
<222>  (6414)..(6414)
<223>  n is a, c, g, or t

<220>
<221>  misc_feature
<222>  (6437)..(6441)
<223>  n is a, c, g, or t

<220>
<221>  misc_feature
<222>  (6458)..(6459)
<223>  n is a, c, g, or t

<220>
<221>  misc_feature
<222>  (6593)..(7103)
<223>  n is a, c, g, or t

<400>  1
gnnnnnnnnn nnnnnnnnnn nnnnnnnnnn nnnnnnnnnn nnnnnnnnnn nnnnnnnnnn      60

nnnnnnnnnn nnnnnnnnnn nnnnnnnnnn nnnnnnnnnn nnnnnnnnnn nnnnnnnnnn     120

nnnnnnnnnn nnnnnnnnnn nnnnnnnnnn nnnnnnnnnn nnnnnnnnnn nnnnnnnnnn     180

nnnnnnnnnn nnnnnnnnnn nnnnnnnnnn nnnnnnnnnn nnnnnnnnnn nnnnnnnnnn     240

nnnnnnnnnn nnnnnnnnnn nnnnnnnnnn nnnnnnnnnn nnnnnnnnnn nnnnnnnnnn     300

nnnnnnnggg gaggagccaa gatggccgaa taggaacagc tccggtctac agctcccagc     360

gtgagcgacg cagaagacgg ntgatnttct gcatttccat ctgaggtacc gggttcatct     420

cactagggag tgccagacag tgggcgcagg ccagtgtgtg tgcgcaccgt gcgcgagccg 480

aagcagggcg aggcattgcc tcacctgggn nnaagcgcaa ggggtcaggg agttcccttt 540

ctgagtcaaa gaaaggggtg acggtcgnca cctggaaaat cgggtcactc ccacccgaat 600

attgcgcttt tcagaccggc ttaagaaacg gcgcaccacg agactatatc ccacacctgg 660

cnntcggagg gtcctacgcc cacggaatct cgctgattgc tagcacagca gtctgagatc 720

aaactgcaag gcggcaacga ggctggggga ggggcgcccg ccattgccca ggcttgctta 780

ggtaaacaaa gcagccgggn aagctcgaac tgggtggagc ccaccacagc tcaaggaggc 840

ctgcctgcct ctgtaggctc cacctctggg ggcagggcac agacaaacaa aaaggcagca 900

gtaacctctg cagacttaag tgtccctgtn ctgacagctt tgaagagagc agtggttctc 960

ccagcacgca gctggagatc tgagaacggg cagacagact gcctcctcaa gtgggtccct 1020

gactcctgac ccccgagcag cctaactggg aggcaccccc cagcaggggc acactgacac 1080

ctcacancgg cagggtattc caacagacct gcagctgagg gtcctgtctg ttagaaggaa 1140

aactaacaac cagnnaaagg acatctacac cgaaaaccca tctgtacatc accatcatca 1200

aagaccaaaa gtagataaaa ccacaaagat ggggaaaaaa cagaacagaa aaactggaaa 1260

ctctaaaacg cagagcgccn tctcctcctc caaaggaacg cagttcctca ccagcaacag 1320

aacaaagctg gatggagaat gnattttgac gagctgagag aagaaggctt cagacgatnn 1380

nnnnnnncaa attactctga gctacgggag gacattcaaa ccaaaggcaa agaagttgaa 1440

aactttgaaa aaaatttaga agaatgtata actagaataa ccaatacaga gaagtgctta 1500

aaggagctga tggagctgaa aaccaaggct cgagaactac gtgaagaatg cagaagcctc 1560

aggagccgat gcgatcaact ggaagaaagg gtatcagcaa tggaagatga aatgaatgaa 1620

atgaagcgan gaagggaagt ttagagaaaa aagaataaaa agaaatgagc aaagcctcca 1680

agaaatatgg gactatgtga aaagaccaaa tctacgtctg attggtgtac ctgaaagtga 1740

tgtggagaat ggaaccaagt tggaaaacac tctgcaggat attatccagg agaacttccc 1800

caatctagca aggcaggcca acgttcagat tcaggaaata cagagaacgc cacaaagata 1860

ctcctcgaga agagcaactc caagacacat aattgtcaga ttcaccaaag ttgaaatgaa 1920

ggaaaaaatg ttaagggcag ccagagagaa aggtcgggtt accctcaaag gaaagcccat 1980

cagactaaca gtggatctct cggcagaaac cctacaagcc agaagagagt gggggccaat 2040

attcaacatt cttaaagaaa agaattttca acccagaatt tcatatccag ccaaactaag 2100

cttcataagt gaannnggag aaataaaata ctttatagac aagcnaaatg ttgagagatt 2160

ttgtcaccac caggcctgcc ctaaannaga gctcnctgaa ggaagcgcta aacatggaaa 2220

ggaacaaccg gtaccagncc gctgcaaaat catgccaaaa tgtaaagacc attgagacta 2280

ggaagaaact gcatcaacta atgagcaaaa tcaccagcta acatcataat gacaggatca 2340

aattcacaca taacaatatt aactttaaat ataaatggac taaattctgc aattaaaaga 2400

cacagactgg caagttggat aaagagtcaa gacccatcag tgtgctgtat tcaggaaacc 2460

catctcnacg ntgcagagac acacataggc tcaaaataaa aggatggagg aagatctacc 2520

aagccaatgg aaaacaaaaa aaggcagggg ttgcaatcct agtctctgat aaaacagact 2580

ttaaaccaac aaagatcaaa agagacaaag aaggccatta cataatggta aagggatcaa 2640

ttcaacaaga ggagctaact atcctaaann nntatttatg cacccaatac aggagcaccc 2700

agattcataa agcaagtcct gagtgaccta caaagagact tagactccca cacattaata 2760

atgggagact ttaacacccc actgtcaaca ttagacagat caacgagaca gaaagtcaac 2820

aaggataccc aggaattgaa ctnncagctc tgcaccaagc agacctanat agacatctac 2880

agaactctcc accccaaatc aacagaatat accttttttt ncagcaccac accacaccta 2940

ttccaaaatt gaccacatag ttggannnag taaagctctc ctcagcaaat gtaaaagaac 3000

agaaattata acaaactatc tctcagacca cagtgcaatc aaactagaac tcaggattaa 3060

gaatctcact caaagccgct caactacatg gaaactgaac aacctgctcc tgnaatgact 3120

actgggtaca taacgaaatg aaggcagaaa taaagatgtt ctttgnnnaa accaacgaga 3180

acaaagacac cacataccag annnatctct gggacgcatt caaagcagtg tgtagaggga 3240

aatttatagc actaaatgcc tacaagagaa agcaggaaag atccaaaatt gacaccctaa 3300

catcacaatt aaaagaacta gaaaagcaag agcaaacaca ttcaaaagct agcagaangg 3360

caagaaataa ctaaaatcag agcagaactg naaggaaata gagacacaaa aaacccttca 3420

aaaaatcaat gaatccagga gctggttttt tgaaaggatc aacaaaattg atagaccgct 3480

agcnaagact aataaagaaa aaaagagaga agaatcaaat agacacaata aaaaatgata 3540

aaggggatat caccaccgat cccacagaaa tacaaactac catcagagaa tactacaaac 3600

acctctacgc aannnataaa ctagaaaatc tagaagaana tggatacatt cctcgacaca 3660

tacactctcc caagactaaa ccaggaagna agttgaatct ctgaatagac caataacagg 3720

ctnctgaaat tgtgnnngca ataatcaata gtttaccaac caaaaagagt ccaggaccag 3780

atggattcac agncnnncga attctaccag aggtacatgg aggaactggt accattcctt 3840

ctgaaactat tccnnnnnnn aatcaataga aaaagaggga atcctccnct aactcatttt 3900

atgaggccag catcattctg ataccaaang ccgggcagag acacaaccaa aaannnnnag 3960

agaatttnta gaccaatatc cttgatgaan nnnncnnnnn nattgatgca aaaatcctca 4020

ataaaatact ggcaaaccga atccattagc acatcaaaaa gcttatccac catgatcaag 4080

tggngcttca tccctgggat gcaaggctgg ttcaatannt acgcaaatca ataaatgtaa 4140

tccagcatat aaancagagc caaagacaaa aaccacatga ttatnnctca atagatgcag 4200

aaaanagcct ttgacaaaat tcaacaaccc ttcatgctaa aaactctcaa taannattag 4260

gtattgatgg gacgtatttc aaaataataa gagctatcta tgacaaaccc acagccaata 4320

tcatactgaa tgggcaaaaa ctggaagcat ntccctttga aaanccggca caagacnagg 4380

gatgccctct ctcaccgctc ctattcnnnn aacatagtgt tgnngaagtt nnctggccag 4440

ggcaatcagg caggagaagg aaataaaggg tattcaatta ggaaaagagg aagtcaaatt 4500

gtccctgttt gcagacgaca tgattgtata tcntagaaaa ccccatcgtc tcagcccaaa 4560

atcntcctta agctgataag caacttcagc aaagtctcag gatacaaaat cnaatgtaca 4620

aaaatcacaa gcattcttat acaccaacaa cagacaaaca gagagccaaa tcatgggnnt 4680

gaacnnnntc ccattcgtaa ttgcnnttca aagagaataa aatacctagg aatccaactt 4740

acaagggatg tgaaggacct cttcaaggag aactacaaac cactgctcaa ggaaataaaa 4800

gaggacacaa acaaatggaa gaacattcca tgctcatggg taggaagaat caatatcgtg 4860

aaaatggcca tactgcccan aggtaattta cagattcaat gccatcccca tcaagctacc 4920

aatgactttc ttcacagaat nnnntggaaa aaactacttt aaagttcata tggaaccaaa 4980

aaagagccnc gcattgccaa gtcaatccta agccaaaaga acaaagctgg aggcatcaca 5040

ctacctgact tcaaacnnta tactacaagg ctacagtaac caaaacagca tggntactgg 5100

taccaaaaca gagatataga tcaatggaac agaacagagc cctcagaaat aatgccgcat 5160

atctacaact atctgatctt tgnacaaacc tgagaaaaac aagcaatggg gaaaggattc 5220

cctatttaat aaatggtgct ggngaaaact ggctagccat atgtagaaag ctgaaactgg 5280

atcccttcct tacaccttat acaaaaatca atnnnntcan nnnagatgga ttaaagattt 5340

aaacgttaaa cctaaaacca taaaannnna ccctagaaga aaacctaggc attaccattc 5400

annggacata ggcgtgggca aggacttcat gtnnnnncca aaacaccaaa agcaatggca 5460

acaaaagaca aaattgacaa atggnnngat ctaattaaac taaagagctt ctgcacagca 5520

aaagaaacta ccatcagagt gaanncaggc aacctacaac atgggagaaa attttcgcaa 5580

cctactcatc tgancaaagg gctaatatcc agaatctaca atgaactcaa acaaatttac 5640

aagaaannna aaacaaacaa ccccatcaaa aagtgggtga aggacatgaa cagacacttc 5700

tcaaaagaag acatttatgc agccaaanaa acacatgaag aaatgctcat catcactggc 5760

catcagagaa atgcaaatca aaaccactat gagatatcat ctcacaccag ttnnagaatg 5820

gcaatcantt aaaaagntca ggaaacaaca ggtgctggag aggatgcgga ngaaatagga 5880

acacttttac actgttggtg ggactgtaaa ctagttcaac cattgtggaa gtcagtgtgg 5940

cnngattcct cagggatcta gnaactagaa ataccatttg acccagccat cccattactn 6000

nnngggtata taccncaaat gagtataaat catgctgnnc tataaagaca catgcacacg 6060

tnnatgttta ttgcggcact atnnnntcan nnnnncaata gcaaagactt ggaaccaacc 6120

cannnaatgt ccaacnaatg atagactgga ttaagaaaat gtggcacata tacaccatgg 6180

annatactat gcagnnnnnc cataaaaaat gatgagttca tatcctttgt agggacatgg 6240

atgaaattgg nnnnnaaann ccatcattct cagtaaacta tcgcaagaac aaaaaaccaa 6300

acaccgcann ntattctcac tcataggtgg gaattgnnaa caantgagat cacatggaca 6360

caggaagggg aatatcacac tctggnnnnn nnnnggactg tggtggggtc gggnggaggg 6420

gggagggata gcattgnnnn nggagatata cctaatgnnc tagatgacac attagtgggt 6480

gcagcgcacc agcatggcac atgtatacat atgtaactaa cctgcacaat gtgcacatgt 6540

accctaaaac ttagagtata ataaaaaaaa aaaaaaaaaa aaaaaaaaaa aannnnnnnn 6600

nnnnnnnnnn nnnnnnnnnn nnnnnnnnnn nnnnnnnnnn nnnnnnnnnn nnnnnnnnnn 6660

nnnnnnnnnn nnnnnnnnnn nnnnnnnnnn nnnnnnnnnn nnnnnnnnnn nnnnnnnnnn 6720

nnnnnnnnnn nnnnnnnnnn nnnnnnnnnn nnnnnnnnnn nnnnnnnnnn nnnnnnnnnn 6780

nnnnnnnnnn nnnnnnnnnn nnnnnnnnnn nnnnnnnnnn nnnnnnnnnn nnnnnnnnnn 6840

nnnnnnnnnn nnnnnnnnnn nnnnnnnnnn nnnnnnnnnn nnnnnnnnnn nnnnnnnnnn 6900

nnnnnnnnnn nnnnnnnnnn nnnnnnnnnn nnnnnnnnnn nnnnnnnnnn nnnnnnnnnn 6960

nnnnnnnnnn nnnnnnnnnn nnnnnnnnnn nnnnnnnnnn nnnnnnnnnn nnnnnnnnnn 7020

nnnnnnnnnn nnnnnnnnnn nnnnnnnnnn nnnnnnnnnn nnnnnnnnnn nnnnnnnnnn 7080

nnnnnnnnnn nnnnnnnnnn nnna 7104

<210> 2
<211> 21
<212> DNA
<213> Homo sapiens

<400> 2
gcactaaatg cctacaagag a 21

<210> 3
<211> 21
<212> DNA
<213> Homo sapiens

<400> 3
gatagaccgc tagcaagact a 21

<210> 4
<211> 21
<212> DNA
<213> Homo sapiens

<400> 4
gaagttgaat ctctgaatag a 21

<210>  5
<211>  21
<212>  DNA
<213>  Homo sapiens

<400>  5
ggacctcttc aaggagaact a                                                21


<210>  6
<211>  21
<212>  DNA
<213>  Homo sapiens

<400>  6
ggagaggatg cggagaaata g                                                21

## Description

### FIELD OF THE INVENTION

The present invention relates to a suppressor or inhibitor of (long interspersed element 1) LINE1 (L1) expression for medical use, particularly for use in the treatment and/or prevention of primary or secondary immunodeficiency, or of pathologies that display an immunosuppressed phenotype, preferably of cancers and/or metastasis, more preferably of lung cancer, even more preferably non-small cells lung carcinoma (NSCLC), or colorectal cancer (CRC), or of viral diseases.

### BACKGROUND TO THE INVENTION

### Transposable Elements (TEs) account for genome evolution and inter-individual genetic variability.

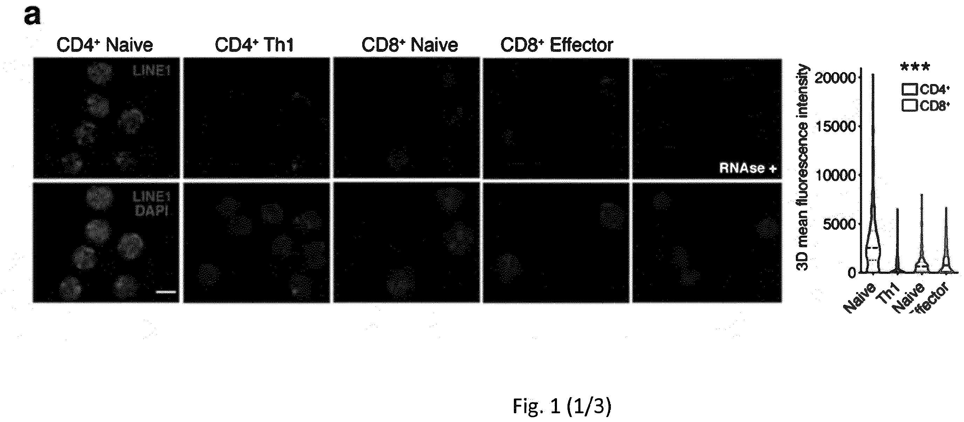

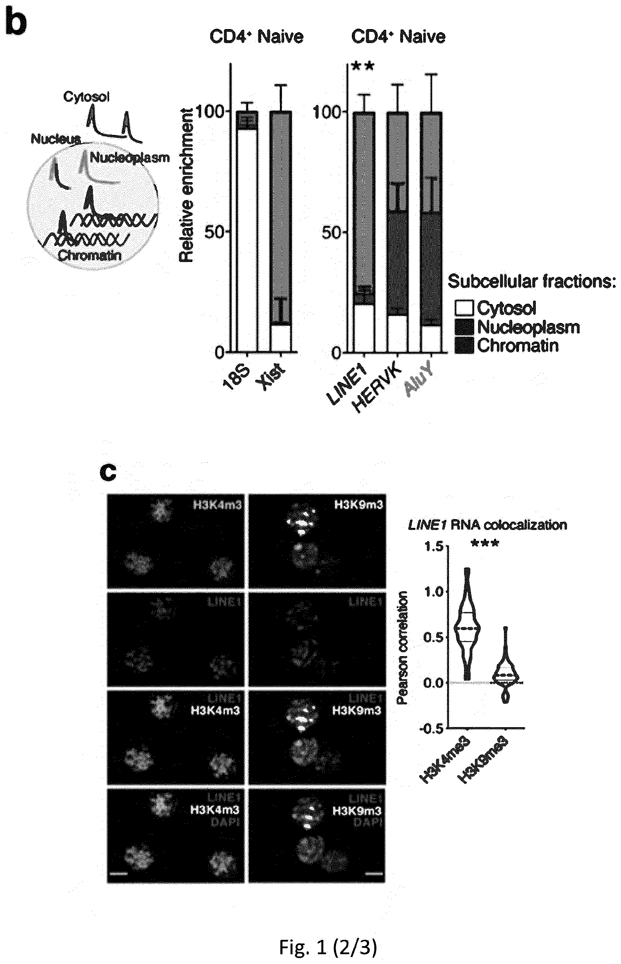

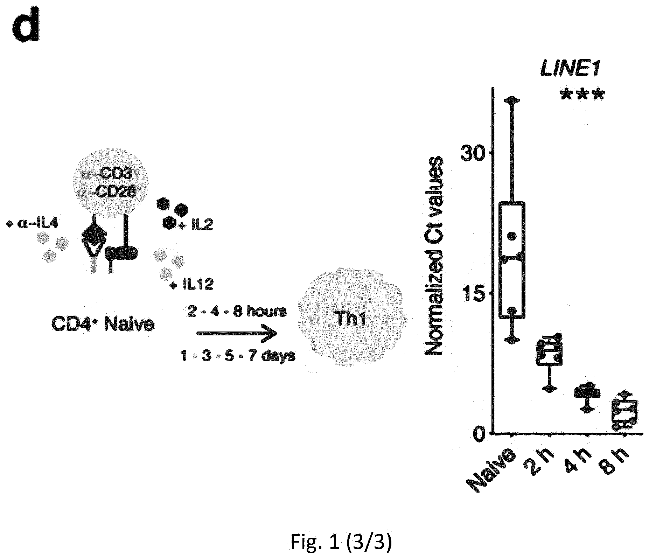

Two thirds of the human genome are constituted of repetitive elements (66 %), among which Transposable Elements (TEs) accounts alone for the 40-45 % of human genome composition **^{1,2}**. One fascinating question for genome biologists is to untangle the functions of this "dark side" of the genome, that still represents an "alive matter" on which evolution can play to generate novel functions. It is clear nowadays that TEs capability of regulating the genome resides mainly in generating a sophisticated plethora of RNA regulatory networks, which in turn influence the transcriptional output of the cell **³⁻⁵**. TEs are organized in four different classes and, with the exception of DNA Transposons, are mainly retrotransposons, which have acquired the ability by using RNA as intermediate to move via a 'copy and paste' mechanism. Retrotransposons include long interspersed elements (LINEs), short interspersed elements (SINEs) and long terminal repeats (LTR) retrotransposons. They are further classified as autonomous or non-autonomous depending on whether they have ORFs that encode for the machinery required for the retrotransposition ⁶. LINE is a class of transposons very ancient and evolutionary successful. Three LINE superfamilies are found in the human genome: LINE1, LINE2 and LINE3, of which only LINE1 is still active. Full-length LINE1 (L1) elements are approximately 6 kb long and constitute an autonomous component of the genome. A LINE1 element has an internal polymerase II promoter and encodes for two open reading frames, ORF1 and ORF2 (Figure 1)⁷. Once the L1 RNA is transcribed, it is exported to cytoplasm for translation, and subsequently assembled with the chaperone RNA-binding proteins ORF1 and the endonuclease and reverse transcriptase ORF2. These ribonucleoparticles are then reimported into the nucleus, where ORF2 makes a single-stranded nick and primes reverse transcription from the 3' end of the L1 RNA. Reverse transcription frequently results in many truncated, nonfunctional insertions and for this reason most of the LINE-derived repeats are short, with an average size around 900-1000 bp. The L1s are estimated to be present in more than 500,000 copies in the human genome ⁷.

The L1 machinery is also responsible for the retrotransposition of the SINEs (which can be classified into three superfamilies: Alu, MIR, MIR3), non-autonomous retroelements without any coding potential, short in length (around 300 bp) and transcribed from polymerase III promoter (Figure 1). The most represented human specific SINE superfamily, the Alu, is represented in 1,090,000 copies in the human genome ⁸.

The LTR retrotransposons are initiated and terminated by long terminal direct repeats embedded by transcriptional regulatory elements. The autonomous LTR retrotransposons contain *gag* and *pol* genes, which encode a reverse transcriptase, integrase, protease and RNAse H (Figure 1). Four superfamilies of LTR exist: ERV- class I, ERV(K) class II, ERV(L) class III, and MalR. MalR is the most represented superfamily of LTR, present in 240,000 copies **⁹**.

Evolutionary biologists hypothesize that self-replicating RNA genomes were the basis of early life on earth, and that the advent of reverse transcription had a pivotal function in the evolution of the first DNA genomes, the more stable deoxyribose-based polymers **^{6,10}**. From this perspective, multiple rounds of reverse transcription could have helped to expand both the size and the complexity of the human genome. It is particularly evident in both mammals and plants that retrotransposons have massively accumulated, driving genome evolution. It is reported that L1 and Alu represent the most prominent catalysts of the human genome evolution ¹¹ and that homologous recombination between TEs could have driven / drives mutations, chromosome rearrangement, deletions, inversions and translocations ¹². TEs are a major source of somatic genomic diversity and interindividual variability ¹³ and TEs insertions have been documented as physiologically occurring ¹⁴⁻¹⁶. In particular L1 retrotransposition has been extensively described to take place in neurons, from fly to man ¹⁷⁻¹⁹, a mechanism that is fine-tuned and epigenetically regulated in neural progenitor development and differentiation, contributing to the somatic diversification of neurons in the brain ^{13,20}. The deregulation of TEs activity is nowadays emerging as an important contributor to many different diseases, as it occurs in neurological, inflammatory and cancer diseases ²¹⁻²³.

The hosts have developed many systems to control TEs expression and expansion ²⁴ (thus, epigenetic modification and noncoding RNAs such Piwi interacting-RNAs) to contain the possible detrimental effects of their retrotransposition. This expansion has achieved a balance between detrimental and beneficial effects, possibly becoming a novel regulatory mechanism to promote genomic functions acquired through evolution ³. It is nowadays accepted, both in mouse and in human, that TEs have been co-opted into multiple regulatory functions for the accommodation of the host genomes metabolisms and transcription, mediated both by their DNA elements and by their transcribed RNAs counterparts.

### Not just transposition: TEs RNAs are a prolific source for novel regulatory functions.

TEs were first discovered in maize by Barbara McClintock almost 80 years ago. She suggested these elements as "controlling elements" able to regulate the genes activity **^{25,26}**. Her theories, even if dismissed for a long time, were pioneering and with the advent of Next Generation Sequencing (NGS) technologies have been thoroughly revised. Currently emerging concept is that TEs interact with the transcriptional regulatory functions of the hosts genomes **^{3,4,27,28}**.

Although a massive portion of the literature has been centered on the study of the retrotransposition and the effects of the *de novo* insertions, it is worth to notice that TEs can have RNA regulatory functions decoupled from their retrotransposition.

International decade long projects as ENCODE (Encyclopedia of DNA Elements) and FANTOM (Functional Annotation of the Mammalian Genome) have produced and bioinformatically analyzed a vast number of datasets opening the way for studying TEs. These results revealed that TEs have precise functions in establishing and influencing the cell type specific transcriptional programs, creating regulatory networks that are fostered both by their genomic elements and the derived transcripts ^{3,28}, revealing that the RNAs transcribed from this elements could have a myriad of functions, definitely changing the way in which many genomic concepts were written in textbooks ²⁹.

These studies clarified that TEs can create novel or alternative promoters **³⁰**, promote the assembly of transcription factors **³¹** and epigenetic modifiers and favor their spreading **³²** and the regulation of gene expression. Further, TEs in particular SINEs and HERVs, have been demonstrated to have function in 3D genome folding, as the binding sites for chromatin organizers **³³⁻³⁵**.

In the 2009 Faulkner et al. **³⁶**, demonstrated for the first time that TEs are widely expressed in human and mouse cell types with tissue-specific patterns of expression, suggesting a specific spatiotemporal activation of retrotransposons. Faulkner et al. further demonstrated that up to the 30% of the transcripts initiate within repetitive elements **³⁶**. It is interesting to notice that tissues of embryonic origin contain the highest proportion of transposable element-derived sequences in their transcriptomes, with specific expression of LTR in placenta and oocytes ³⁷. In accordance, it was recently found that different classes of repeats are specifically enriched in genes with a definite spatiotemporal expression, further dictating their timing and magnitude of expression in development ³⁸.

Within this scenario, TEs magnify the transcriptome complexity in different ways: generating antisense transcripts, usually in proximity to gene promoters **³⁶**; acting on the maturation of mRNAs via nursing alternative splicing sites for tissue specific exonization **^{39,40}** and providing alternative polyadenylation signals **^{41,42}** and sites for the RNA-mediated decoy ⁴³. Furthermore, TEs contribute to RNA regulatory sequences within introns and untranslated regions (UTRs) **³⁶**. It is important to notice that TEs are major contributors to long noncoding RNAs (IncRNAs) **^{44,45}**. In this scenario, an enhancer RNAs function was proposed for LTR derived transcripts, as required for pluripotency maintenance in mouse and human embryonic stem (ES) cells **^{46,47}**. Further, it has been demonstrated that LINEs and SINEs are expressed as RNAs tightly associated to the chromatin compartment, where they localized at euchromatin, suggesting a possible function of these RNAs in 3D genome folding **⁴⁸**. L1s have been described also as chromatin associated RNAs both in embryogenesis, regulating open chromatin accessibility **^{49,50}**, and in mouse ES cells, where they are involved in the regulation of genes required for cell identity maintenance and 2-cell stage differentiation **⁵¹**.

Although these seminal papers have increased the consciousness and the knowledge on functions of TEs, highlighting important epigenetic roles for transposons in embryogenesis and development, contribution of TEs to adult cell plasticity and diseases occurrence and progression is still poorly investigated. This as a result of the intrinsic difficulties in studying TEs, which due to their repetitive nature, high degree of homology, sequence divergence and degeneration render almost unfeasible the application of the technologies established for biallelic genes, in particular in bioinformatic.

### Relevance of studying T cell transcriptional plasticity within Tumor microenvironment

It is nowadays well demonstrated that innate and adaptive immune responses play a fundamental role in tumorigenesis; the interplay between tumor cells and immune system is defined as cancer immunoediting. Indeed, the most complex form of immunoediting is represented by the crosstalk between tumor infiltrating T lymphocytes (TILs) and tumor cells, that expose neo-antigens on their surface within the tumor microenvironment; this could result in either tumor elimination, equilibrium between immune response and residual tumor cell growth, or tumor escape from immune control ⁵².

Tumor microenvironment can be very heterogenous in terms of the immune infiltrate abundance, composition and response ⁵³; in particular, the relative abundance and effector functions of TILs can be inhibited by the development of a tumor specific transcriptional program able to disempower, exclude and evade the immune system ⁵⁴. The tumor-dependent immunosuppressive mechanisms rely on a complex network that establishes within the tumor microenvironment and is based on the upregulation of modulatory molecules, collectively called immune checkpoints, whose function is only partially characterized ⁵⁵. Nevertheless, these molecules (e.g. CTLA-4, PD-1, PDL-1) are target of immune checkpoint inhibitors (ICIs) therapy (immunotherapy), that unleashes the spontaneous anti-tumor immune responses in such a powerful way that it has created a paradigm shift in cancer therapy ⁵⁶⁻⁵⁸. However, while it is quite clear that tumor types that are more antigenic because of the high mutational load (e.g., melanoma, lung, kidney, bladder) are more likely to respond to immunotherapy, it is less clear as to why most patients with these highly antigenic tumors do not have a durable response or do not respond at all to immunotherapy; indeed, the fraction of patients that do not respond remains high, and the efforts in the field are mainly focused on searching specific ICIs against novel surface markers expressed in T cells subsets, also defined at single cell level ^{54,59-62}. Almost nothing is reported regarding the genomic and epigenetic mechanisms that govern the intratumoral dysfunctional state of TILs, with the aim to reestablish their function, acting on reversible mechanisms of transcriptional plasticity.

Inventors will characterize two most frequent types of human cancer where immunotherapy is more (non small cells lung carcinoma, NSCLC) or less (colorectal cancer, CRC) frequently used and effective. They are the first and second causes of death worldwide, respectively. Lung cancer is the most common cancer in terms of incidence (2.09 million cases estimated in 2018)⁶³, with NSCLC accounting for 84% of lung tumor cases ⁶⁴, with an overall survival at five years up to 19%. Colorectal cancer (CRC) is the third most common cancer, accounting for 1.84 million estimated new cases in 2018, with a 60% overall survival at five years ^{63,65}.

In order to define the molecular parameters that could predict the TILs immune responsiveness, inventors will perform transcriptome analyses by RNA-seq and chromatin RNA-seq, profiling CD4+ and CD8+ T effectors and regulatory CD4+ T cells.

Inventors have found that these TEs containing transcripts represent novel therapeutic targets, unpredictable with another strategies, for promoting TILs transcriptional reshape leading to unleashed effector immune response.

### BRIEF DESCRIPTION OF THE INVENTION

It is therefore an object of the invention a suppressor or inhibitor of (Long Interspersed Nuclear Element 1) LINE1 (L1) expression for medical use.

Preferably the suppressor or inhibitor of LINE1 expression is for use in the treatment and/or prevention of primary or secondary immunodeficiency, or of pathologies that display an immunosuppressed phenotype, preferably of cancers and/or metastasis, more preferably of lung cancer, even more preferably non-small cells lung carcinoma (NSCLC), or colorectal cancer (CRC), or of viral diseases such as immunodeficiencies due to HIV, Lymphocytic choriomeningitis virus (LCMV).

Preferably the suppressor or inhibitor is at least one molecule selected from the group consisting of:
a) an antibody or a fragment thereof;
b) a polypeptide;
c) a small molecule;
d) a polynucleotide coding for said antibody or polypeptide or a functional derivative thereof;
e) a polynucleotide, such as antisense construct, antisense oligonucleotide, RNA interference construct or siRNA,
e) a vector comprising or expressing the polynucleotide as defined in d) or e);
f) a CRISPR/Cas9 component, e.g. a sgRNA;
g) a host cell genetically engineered expressing said polypeptide or antibody or comprising the polynucleotide as defined in d) or e) or at least one component of f).

In a preferred embodiment the polynucleotide is an isolated inhibitory nucleic acid targeting LINE1. Preferably the inhibitory nucleic acid comprises a sequence of nucleotides that are identical or complementary to 5, 6, 7, 8 or 9 to 100, preferably to 10 to 50, consecutive nucleotides of SEQ ID NO: 1.

Preferably said inhibitory nucleic acid is at least one RNA inhibitor, preferably selected from the group consisting of: antisense oligo (ASO), gapmer, mixmer, shRNA, siRNA, stRNA, snRNA, more preferably said inhibitory nucleic acid is modified, such as2'-deoxy-2'-fluoro-β-D-arabinonucleid acid (FANA) ASO, and/or comprises one or more modified bonds or bases. Preferably said ASO is designed on a consensus sequence of L1, especially L1M subfamilies and ORF2-3'UTR region.

Preferably the ASO comprises a sequence capable of hybridizing or complementary to the ORF2-3'UTR region of L1, preferably of specific L1 subfamilies, such as L1M subfamilies.

Preferably the ASO comprises a nucleic acid sequence that targets one of the following sequences:
LINE1-a GCACTAAATGCCTACAAGAGA (SEQ ID NO:2)
LINE1-b GATAGACCGCTAGCAAGACTA (SEQ ID NO:3)
LINE1-c GAAGTTGAATCTCTGAATAGA (SEQ ID NO:4)
LINE1-d GGACCTCTTCAAGGAGAACTA (SEQ ID NO:5)
LINE1-e GGAGAGGATGCGGAGAAATAG (SEQ ID NO:6).
or the corresponding RNA sequence.

The suppressor or inhibitor for use according to the invention may be used in T cells, preferably CD4+ T naive cells or a CD8+ T cell, Tumor infiltrating Lymphocytes TILs both CD4+ and CD8+, B cells, Natural Killer cells or Tumor cells.

The suppressor or inhibitor for use according to the invention may be used in combination with an immunotherapy and/or with a radiotherapy and/or chemotherapeutic agent and/or with targeted therapies which promote raising of new antigens and immunity response and/or with immunity system adjuvants, preferably said immunotherapy comprises administration of an immune checkpoint inhibitor and/or chimeric antigen receptor (CAR)-expressing immune effector cells, preferably the immune checkpoint inhibitor is an or comprises one or more anti-CD137 antibodies; anti-PD-1 (programmed cell death 1) antibodies; anti-PDLl (programmed cell death ligand 1) antibodies; anti-PDL2 antibodies; or anti-CTLA-4 antibodies.

The suppressor or inhibitor for use according to the invention may be used in Adoptive cell transfer (ACT), cell therapy treatment, mismatched bone marrow transplantation, mismatched NK cell infusion or cytokine-induced killer (CIK) cell infusion, or wherein said suppressor or inhibitor is injected in the tumour site, e.g. in intestine tumour, melanoma, or delivered by nanoparticles specifically to the site of interest.

Another object of the invention is a pharmaceutical composition comprising the suppressor or inhibitor as defined above and at least one pharmaceutically acceptable carrier, and optionally further comprising a therapeutic agent.

A further object of the invention is a method to modulate the commitment of naive CD4+ T naive cells towards any effector lineage and to modulate the effector response in dysfunctional T cells comprising the step of inhibiting LINE1 expression in said cells, wherein the step of inhibiting LINE1 expression in said cells is performed by means of at least one suppressor or inhibitor as defined above.

Another object of the invention is an isolated human T cell, B cell, NK cell or Tumor cell, wherein said cell is stably or transiently affected in the expression of LINE1, preferably said cell is a CD4+ T naive cell or a CD8+ T cell, or a dysfunctional T cell, e.g. a TIL.

A further object of the invention is a composition comprising at least one cell or combinations thereof as defined above, said composition preferably further comprising at least one physiologically acceptable carrier.

Preferably the cell or the composition as above defined is for use as a medicament, preferably for use in the treatment and/or prevention of primary or secondary immunodeficiency, or of pathologies that display an immunosuppressed phenotype, preferably of cancers and/or metastasis, more preferably of lung cancer, even more preferably non-small cells lung carcinoma (NSCLC), or colorectal cancer (CRC), or of viral diseases such as immunodeficiencies due to HIV, Lymphocytic choriomeningitis virus (LCMV).

Preferably said cell or composition is used in Adoptive cell transfer (ACT), cell therapy treatment, mismatched bone marrow transplantation, mismatched NK cell infusion or cytokine-induced killer (CIK) cell infusion, or wherein said cell or composition is injected in the tumour site, e.g. in intestine tumour, melanoma, or delivered by nanoparticles specifically to the site of interest.

### DETAILED DESCRIPTION OF THE INVENTION

By the term "suppressor or inhibitor" or a "molecule which (selectively) suppresses or inhibits" it is meant a molecule that effects a change in the expression of the target. The change is relative to the normal or baseline level of expression in the absence of the "suppressor or inhibitor" or of the molecule, but otherwise under similar conditions, and it represent a decrease in the normal/baseline expression. The suppression or inhibition of the expression of the target may be assessed by any means known to the skilled in the art. The assessment of the expression level or of the presence of the target is preferably performed using classical molecular biology techniques such as (real time Polymerase Chain Reaction) qPCR, microarrays, bead arrays, RNAse protection analysis or Northern blot analysis or cloning and sequencing. In the context of the present invention, the target is the gene, the mRNA, the cDNA, or the encoded protein thereof. The above described molecules also include salts, solvates or prodrugs thereof. The above described molecules may be or not solvated by H₂0. In the context of the present invention the term "targeting" or "complementary" may be intended as being fully or partly complementary to all of or part of the target sequence or as being capable of hybridizing to all or part of specific target sequence.

The polynucleotides as above described, as e.g. the siRNAs, may further comprise dTdT or UU 3'-overhangs, and/or nucleotide and/or polynucleotide backbone modifications as described elsewhere herein. In the context of the present invention, the term "polynucleotide" includes DNA molecules (e.g., cDNA or genomic DNA) and RNA molecules (e.g., mRNA, siRNA, shRNA) and analogs of the DNA or RNA generated using nucleotide analogs. The polynucleotide may be single-stranded or double-stranded. The RNA inhibitors as above defined are preferably capable of hybridizing to all or part of specific target sequence. Therefore, RNA inhibitors may be fully or partly complementary to all of or part of the target sequence. The RNA inhibitors may hybridize to the specified target sequence under conditions of medium to high stringency. An RNA inhibitor may be defined with reference to a specific sequence identity to the reverse complement of the sequence to which it is intended to target. The antisense sequences will typically have at least about 75%, preferably at least about 80%, at least about 85%, at least about 90%, at least about 95% or at least about 99% sequence identity with the reverse complements of their target sequences.

The term polynucleotide and polypeptide also include derivatives and functional fragments thereof. The polynucleotide may be synthesized using oligonucleotide analogs or derivatives (e.g., inosine or phosphorothioate nucleotides).

The molecule according to the invention may be an antibody or derivatives thereof.

The term gene herein also includes corresponding orthologous or homologous genes, isoforms, variants, allelic variants, functional derivatives, functional fragments thereof. The expression "protein" is intended to include also the corresponding protein encoded from a corresponding orthologous or homologous genes, functional mutants, functional derivatives, functional fragments or analogues, isoforms thereof.

In the context of the present invention, the term "polypeptide" or "protein" includes:
i. the whole protein, allelic variants and orthologs thereof;
ii. any synthetic, recombinant or proteolytic functional fragment;
iii. any functional equivalent, such as, for example, synthetic or recombinant functional analogues.

In the present invention "functional mutants" of the protein are mutants that may be generated by mutating one or more amino acids in their sequences and that maintain their activity. Indeed, the protein of the invention, if required, can be modified in vitro and/or in vivo, for example by glycosylation, myristoylation, amidation, carboxylation or phosphorylation, and may be obtained, for example, by synthetic or recombinant techniques known in the art. The term "derivative" as used herein in relation to a protein means a chemically modified peptide or an analogue thereof, wherein at least one substituent is not present in the unmodified peptide or an analogue thereof, i.e. a peptide which has been covalently modified. Typical modifications are amides, carbohydrates, alkyl groups, acyl groups, esters and the like. As used herein, the term "derivatives" also refers to longer or shorter polypeptides having e.g. a percentage of identity of at least 41 % , preferably at least 41.5%, 50 %, 54.9% , 60 %, 61.2%, 64.1%, 65 %, 70 % or 75%, more preferably of at least 85%, as an example of at least 90%, and even more preferably of at least 95% with the herein disclosed genes and sequences, or with an amino acid sequence of the correspondent region encoded from orthologous or homologous gene thereof. The term "analogue" as used herein referring to a protein means a modified peptide wherein one or more amino acid residues of the peptide have been substituted by other amino acid residues and/or wherein one or more amino acid residues have been deleted from the peptide and/or wherein one or more amino acid residues have been deleted from the peptide and or wherein one or more amino acid residues have been added to the peptide. Such addition or deletion of amino acid residues can take place at the N-terminal of the peptide and/or at the C-terminal of the peptide. A "derivative" may be a nucleic acid molecule, as a DNA molecule, coding the polynucleotide as above defined, or a nucleic acid molecule comprising the polynucleotide as above defined, or a polynucleotide of complementary sequence. In the context of the present invention the term "derivatives" also refers to longer or shorter polynucleotides and/or polynucleotides having e.g. a percentage of identity of at least 41 % , 50 %, 60 %, 65 %, 70 % or 75%, more preferably of at least 85%, as an example of at least 90%, and even more preferably of at least 95% or 100% with e.g. SEQ ID NO: 1-6 or with their complementary sequence or with their DNA or RNA corresponding sequence. The term "derivatives" and the term "polynucleotide" also include modified synthetic oligonucleotides. The modified synthetic oligonucleotide are preferably LNA (Locked Nucleic Acid), phosphoro-thiolated oligos or methylated oligos, morpholinos, 2'-O-methyl, 2'-O-methoxyethyl oligonucleotides and cholesterol-conjugated 2'-O-methyl modified oligonucleotides (antagomirs). The term "derivative" may also include nucleotide analogues, i.e. a naturally occurring ribonucleotide or deoxyribonucleotide substituted by a non-naturally occurring nucleotide. The term "derivatives" also includes nucleic acids or polypeptides that may be generated by mutating one or more nucleotide or amino acid in their sequences, equivalents or precursor sequences. The term "derivatives" also includes at least one functional fragment of the polynucleotide.In the context of the present invention "functional" is intended for example as "maintaining their activity". As used herein "fragments" refers to polynucleotides having preferably a length of at least 1000 nucleotides, 1100 nucleotide, 1200 nucleotides, 1300 nucleotides, 1400 nucleotides, 1500 nucleotides or to polypeptide having preferably a length of at least 50 aa, 100 aa, 150 aa, 200 aa, 250 aa, 300 aa. The term "polynucleotide" also refers to modified polynucleotides. As used herein, the term "vector" refers to an expression vector, and may be for example in the form of a plasmid, a viral particle, a phage, etc. Such vectors may include bacterial plasmids, phage DNA, baculovirus, yeast plasmids, vectors derived from combinations of plasmids and phage DNA, viral DNA such as vaccinia, adenovirus, lentivirus, fowl pox virus, and pseudorabies. Large numbers of suitable vectors are known to those of skill in the art and are commercially available. The polynucleotide sequence, preferably the DNA sequence in the vector is operatively linked to an appropriate expression control sequence(s) (promoter) to direct mRNA synthesis. As representative examples of such promoters, one can mention prokaryotic or eukaryotic promoters such as CMV immediate early, HSV thymidine kinase, early and late SV40, LTRs from retrovirus, and mouse metallothionein-I. The expression vector may also contain a ribosome binding site for translation initiation and a transcription vector. The vector may also include appropriate sequences for amplifying expression. In addition, the vectors preferably contain one or more selectable marker genes to provide a phenotypic trait for selection of transformed host cells such as dihydro folate reductase or neomycin resistance for eukaryotic cell culture, or such as tetracycline or ampicillin resistance in E. coli. As used herein, the term "host cell genetically engineered" relates to host cells which have been transduced, transformed or transfected with the polynucleotide or with the vector described previously. As representative examples of appropriate host cells, one can cite bacterial cells, such as E. coli, Streptomyces, Salmonella typhimurium, fungal cells such as yeast, insect cells such as Sf9, animal cells such as CHO or COS, plant cells, etc. The selection of an appropriate host is deemed to be within the scope of those skilled in the art from the teachings herein. Preferably, said host cell is an animal cell, and most preferably a human cell. The introduction of the polynucleotide or of the vector described previously into the host cell can be effected by method well known from one of skill in the art such as calcium phosphate transfection, DEAE-Dextran mediated transfection, electroporation, lipofection, microinjection, viral infection, thermal shock, transformation after chemical permeabilization of the membrane or cell fusion. The polynucleotide may be a vector such as for example a viral vector. The polynucleotides as above defined can be introduced into the body of the subject to be treated as a nucleic acid within a vector which replicates into the host cells and produces the polynucleotides. Suitable administration routes of the pharmaceutical composition of the invention include, but are not limited to, oral, rectal, transmucosal, intestinal, enteral, topical, suppository, through inhalation, intrathecal, intraventricular, intraperitoneal, intranasal, intraocular, parenteral (e.g., intravenous, intramuscular, intramedullary, and subcutaneous), chemoembolization. Other suitable administration methods include injection, viral transfer, use of liposomes, e.g. cationic liposomes, oral intake and/or dermal application. In certain embodiments, a pharmaceutical composition of the present invention is administered in the form of a dosage unit (e.g., tablet, capsule, bolus, etc.). For pharmaceutical applications, the composition may be in the form of a solution, e.g. an injectable solution, emulsion, suspension or the like. The carrier may be any suitable pharmaceutical carrier. Preferably, a carrier is used which is capable of increasing the efficacy of the molecules to enter the target cells. Suitable examples of such carriers are liposomes. In the pharmaceutical composition according to the invention, the suppressor or inhibitor may be associated with other therapeutic agents. The pharmaceutical composition can be chosen on the basis of the treatment requirements. Such pharmaceutical compositions according to the invention can be administered in the form of tablets, capsules, oral preparations, powders, granules, pills, injectable, or infusible liquid solutions, suspensions, suppositories, preparation for inhalation. A reference for the formulations is the book by Remington ("Remington: The Science and Practice of Pharmacy", Lippincott Williams & Wilkins, 2000). The expert in the art will select the form of administration and effective dosages by selecting suitable diluents, adjuvants and / or excipients. Pharmaceutical compositions of the present invention may be manufactured by processes well known in the art, e.g., using a variety of well-known mixing, dissolving, granulating, levigating, emulsifying, encapsulating, entrapping or lyophilizing processes. The compositions may be formulated in conjunction with one or more physiologically acceptable carriers comprising excipients and auxiliaries which facilitate processing of the active compounds into preparations which can be used pharmaceutically. Proper formulation is dependent upon the route of administration chosen. Parenteral routes are preferred in many aspects of the invention. For injection, including, without limitation, intravenous, intramusclular and subcutaneous injection, the compounds of the invention may be formulated in aqueous solutions, preferably in physiologically compatible buffers such as physiological saline buffer or polar solvents including, without limitation, a pyrrolidone or dimethylsulfoxide. The compounds are preferably formulated for parenteral administration, e.g., by bolus injection or continuous infusion. Useful compositions include, without limitation, suspensions, solutions or emulsions in oily or aqueous vehicles, and may contain adjuncts such as suspending, stabilizing and/or dispersing agents. Pharmaceutical compositions for parenteral administration include aqueous solutions of a water-soluble form, such as, without limitation, a salt of the active compound. Additionally, suspensions of the active compounds may be prepared in a lipophilic vehicle. Suitable lipophilic vehicles include fatty oils such as sesame oil, synthetic fatty acid esters such as ethyl oleate and triglycerides, or materials such as liposomes. Aqueous injection suspensions may contain substances that increase the viscosity of the suspension, such as sodium carboxyl ethyl cellulose, sorbitol, or dextran. Optionally, the suspension may also contain suitable stabilizers and/or agents that increase the solubility of the compounds to allow for the preparation of highly concentrated solutions. Alternatively, the active ingredient may be in powder form for constitution with a suitable vehicle, e.g., sterile, pyrogen-free water, before use. For oral administration, the compounds can be formulated by combining the active compounds with pharmaceutically acceptable carriers well-known in the art. Such carriers enable the compounds of the invention to be formulated as tablets, pills, lozenges, dragees, capsules, liquids, gels, syrups, pastes, slurries, solutions, suspensions, concentrated solutions and suspensions for diluting in the drinking water of a patient, premixes for dilution in the feed of a patient, and the like, for oral ingestion by a patient. Useful excipients are, in particular, fillers such as sugars, including lactose, sucrose, mannitol, or sorbitol, cellulose preparations such as, for example, maize starch, wheat starch, rice starch and potato starch and other materials such as gelatin, gum tragacanth, methyl cellulose, hydroxypropyl- methylcellulose, sodium carboxy- methylcellulose, and/or polyvinylpyrrolidone (PVP). For administration by inhalation, the molecules of the present invention can conveniently be delivered in the form of an aerosol spray using a pressurized pack or a nebulizer and a suitable propellant. The moelcules may also be formulated in rectal compositions such as suppositories or retention enemas, using, e.g., conventional suppository bases such as cocoa butter or other glycerides. In addition to the formulations described previously, the compounds may also be formulated as depot preparations. Such long acting formulations may be administered by implantation (for example, subcutaneously or intramuscularly) or by intramuscular injection. The compounds of this invention may be formulated for this route of administration with suitable polymeric or hydrophobic materials (for instance, in an emulsion with a pharmacologically acceptable oil), with ion exchange resins, or as a sparingly soluble derivative such as, without limitation, a sparingly soluble salt. Additionally, the compounds may be delivered using a sustained-release system, such as semi-permeable matrices of solid hydrophobic polymers containing the therapeutic agent. Various sustained-release materials have been established and are well known by those skilled in the art. A therapeutically effective amount refers to an amount of compound effective to prevent, alleviate or ameliorate the protein conformational disease. Determination of a therapeutically effective amount is well within the capability of those skilled in the art, especially in light of the disclosure herein. Generally, the amount used in the treatment methods is that amount which effectively achieves the desired therapeutic result in mammals. In particular, the molecules administration should follow the current clinical guidelines. A suitable daily dosage will range from 0.001 to 10 mg / kg body weight, in particular 0.1 to 5 mg / kg. In the case of polynucleotides, a suitable daily dosage may be in the range of 0.001 pg/kg body weight to 10 mg/kg body weight. Typically, the patient doses for parenteral administration of the molecules described herein range from about 1 mg/day to about 10,000 mg/day, more typically from about 10 mg/day to about 1,000 mg/day, and most typically from about 50 mg/day to about 500 mg/day. The range set forth above is illustrative and those skilled in the art will determine the optimal dosing of the compound selected based on clinical experience and the treatment indication.

As used herein, "oligonucleotides" or "polynucleotide" shall mean single or double stranded RNA or DNA, including ASOs and siRNA capable of binding to complementary single or double stranded RNA or DNA target sequences. The sequence-specific portion of the therapeutic oligonucleotides that are ASOs or siRNA of the invention comprise nucleotide sequences of from about 7 bases to about 45 bases in length. Additional bases that are not sequence-specific may be included in the oligonucleotides, such as for example linker sequence. By sequence-specific is meant the portion of the oligonucleotide that is complementary to the target RNA or DNA and/or directs cleavage of the target RNA or DNA.

As used herein, "ASOs" shall mean short stretches (about 7 to about 45 sequence-specific nucleotides) of DNA or derivatized DNA (e.g., phosphorothioated DNA) that contains sequence which is complementary to a target DNA or RNA. The complementary portion of the ASOs will typically range from about 30% to about 100% of the oligonucleotide.

As used herein, "siRNA" shall mean an RNA duplex in which each strand of the duplex contains between about 15 and about 30 bases in length, and wherein at least one of the strands shares at least about 90%, more preferably up to about 100% homology with a DNA or RNA target.

As used herein, "gene expression" shall mean mRNA synthesis or mRNA translation.

In one embodiment of the invention, therapeutic oligonucleotides of the invention are ASOs. ASOs encompass single-stranded DNA or RNA that is complementary to a portion of a specific RNA sequence, or alternatively the complementary gene sequence, and reduce or inhibit gene expression. Non-limiting examples of ASOs include RNA sequences complementary to an mRNA transcript, thereby forming an RNA duplex resulting in reduced levels of translation. Alternatively, an ASO may encompass a DNA sequence complementary to an mRNA transcript, which hybridizes with the mRNA transcript and serves as a substrate for RNaseH.

The technology of antisense oligonucleotides has been known in the art as a promising source of therapeutics. Antisense oligonucleotides rely upon Watson-Crick base pairing between a known nucleic acid sequence and its reverse complement to inhibit gene expression (Jen, K., et al, Stem Cells, 18:307-19 (2000)). Antisense oligonucleotide therapy can be utilized to combat a wide range of disorders, for example the expression of human genes involved in diseases or disorders, or alternatively by targeting the replication of infectious agents (Tanaka, M., et al, Respir. Res., 2:5-9 (2000); Bunnell, B.A., et al, Clin. Micro. Rev., 11:42-56 (1998)). Crucial considerations which must be addressed when designing antisense oligonucleotide therapies include antisense stability in vivo, effective delivery of the antisense oligonucleotide therapeutic, and efficient intracellular localization of the antisense oligonucleotide (Jen, K., et al, Stem Cells, 18:307-19 (2000)).

It is well known that, depending on the target gene, ASOs which hybridize to any part of the target gene, such as coding regions, introns, the 5' untranslated region (5'UTR), translation initiation site, or 3'UTR may have therapeutic utility. Therefore, the sequences listed herein are merely exemplary of the possible therapeutic oligonucleotides that may be used with the invention, which include all of the ASOs known in the art. Furthermore, all of the alternative nucleic acid chemistries proposed in the art can be used with the invention although the degree of effectiveness may vary. Chemistries applicable with the therapeutic oligonucleotides of the invention are discussed in further detail in the section entitled "Conjugation Chemistry and Carrier Molecules" provided infra. In short, the compounds listed herein represent the broad class of therapeutic oligonucleotides of various chemistries which are useful with this invention. In one embodiment of the invention, the sequence-binding portion of ASO and siRNA therapeutic oligonucleotides of the invention is about 7 to about 45 bases in length. In a preferred embodiment of the invention, the sequence-binding portion of ASO and siRNA therapeutic oligonucleotides of the invention is about 10 to about 30 nucleotides in length. In a particularly preferred embodiment of the invention, the sequence-binding portion of ASO and siRNA therapeutic oligonucleotides of the invention is about 15 to about 25 nucleotides in length. Additional oligonucleotides which are useful in the invention include oligonucleotides previously demonstrating efficacy in free form in the art. Therapeutic oligonucleotides of the invention also encompass siRNA. siRNA derive from RNA interference, which is a natural cellular process for silencing the transcription of certain genes (Sharp, P.A., Genes & Dev., 15:485-490 (2001); Carmichael, G.G., Nature, 418:379-380 (2002)). siRNA associate with cellular protein complexes and direct cleavage of complementary target RNAs by those protein complexes.

In the present invention, siRNA encompass duplex RNAs of approximately 15-30 bases in length, one strand of the duplex RNA preferably having at least about 90% homology with a RNA target, more preferably having up to about 100% homology with a RNA target. Alternatively, siRNAs share enough homology with a RNA target to direct cleavage of complementary target RNA by protein complexes. Homology between two nucleotide sequences can be determined by one of ordinary skill in the art using search-based computer programs, such as the BLAST or FASTA programs. Alternatively, one of ordinary skill in the art can determine sequence homology using sequence alignment programs such as MegAlign (contained within the DNASTAR suite of computer programs).

siRNAs are modified with chemical reactive groups described infra, enabling the formation of covalent bonds with mobile proteins, preferably human serum albumin, in a preferred embodiment of the invention, modification of the siRNA duplex through addition of a chemical reactive group occurs at a terminus. Chemical modification of the RNA duplex with a chemical reactive group may occur at any of the 4 termini of the RNA duplex, either the 5' or 3' termini of either of the two RNA strands of the RNA duplex.

Preferably, the inhibitory nucleic acid comprises one or more peptide nucleic acid (PNA) or locked nucleic acid (LNA) molecules or the inhibitory nucleic acid is a ribonucleic acid analogue comprising a ribose ring having a bridge between its 2'-oxygen and 4'-carbon.

Preferably, the ribonucleic acid analogue comprises a methylene bridge between the 2'-oxygen and the 4'-carbon.

Preferably, at least one nucleotide of the inhibitory nucleic acid comprises a modified sugar moiety selected from a 2'-0- methoxyethyl modified sugar moiety, a 2'-methoxy modified sugar moiety, a 2'-0- alkyl modified sugar moiety, and a bicyclic sugar moiety.

Preferably the inhibitory nucleic comprises at least one modified internucleoside linkage selected from phosphorothioate, phosphorodithioate, alkylphosphonothioate, phosphoramidate, carbamate, carbonate, phosphate triester, acetamidate, carboxymethyl ester, and combinations thereof.

L1 subfamily comprises: HAL1,HAL1B, HAL1M8, IN25, L1, L1HS, L1M1_5, L1M1B_5, L1M2A_5, L1M3A 5, L1M3B_5, L1M3C_5, L1M3D_5, L1M3DE 5, L1M4B, L1M6_5end, L1M6B_5end, L1M7_5end, L1MA1, L1MA10, L1MA2, L1MA3, L1MA4, L1MA4A, L1MA5 L1MA5A, L1MA6, L1MA7, L1MA8, L1MA9, L1MB1, L1MB2, L1MB3, L1MB3_5, L1MB4 L1MB5, L1MB6_5, L1MB7, L1MB8, L1MC1, L1MC2, L1MC4, L1MCB_5, L1MD1, L1MD2, L1MDB_5, L1ME_ORF2, L1ME1, L1ME2, L1ME3, L1ME3A, L1ME4A, L1MEA_5, L1MEC_5, L1MED_5, L1MEf_5end, L1PA10, L1PA11, L1PA12, L1PA12_5, L1PA13, L1PA13_5, L1PA14, L1PA15, L1PA16, L1PA2, L1PA3, L1PA4, L1PA5, L1PA6, L1PA7, L1PA8, L1PB1, L1PB2, L1PB2c, L1PB3, L1PB4, L1PREC1, L1PREC2. (https://www.girinst.org/repbase/, Kenji K. Kojima, Human transposable elements in Repbase: genomic footprints from fish to humans, Mob DNA. 2018; 9: 2).

In the context of the present invention, cancer or tumour may include any time of cancer or tumours, e.g. lung cancer, preferably non-small cells lung carcinoma (NSCLC), colorectal cancer (CRC), intestine tumour or melanoma.

The invention will be now illustrated by means of non-limiting examples referring to the following figures.
**Figure 1****.** Characterization of L1 RNAs expression in human T lymphocytes. a) L1 RNAs are specifically expressed in CD4+ naive T cells as probed by RNA FISH for L1. b) L1 RNAs are chromatin associated in CD4+ naive T cells, as assessed by biochemical fractionations followed by RT-qPCR analysis. c) L1 RNAs are enriched in the euchromatin compartment. d) L1 RNAs are promptly downregulated upon T cell activation, as revealed by RT-qPCR analysis.

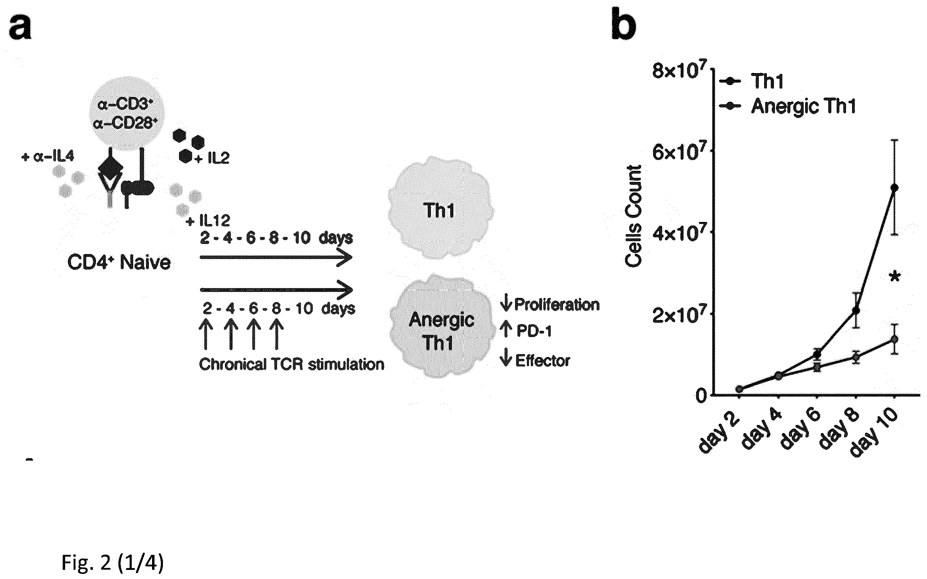

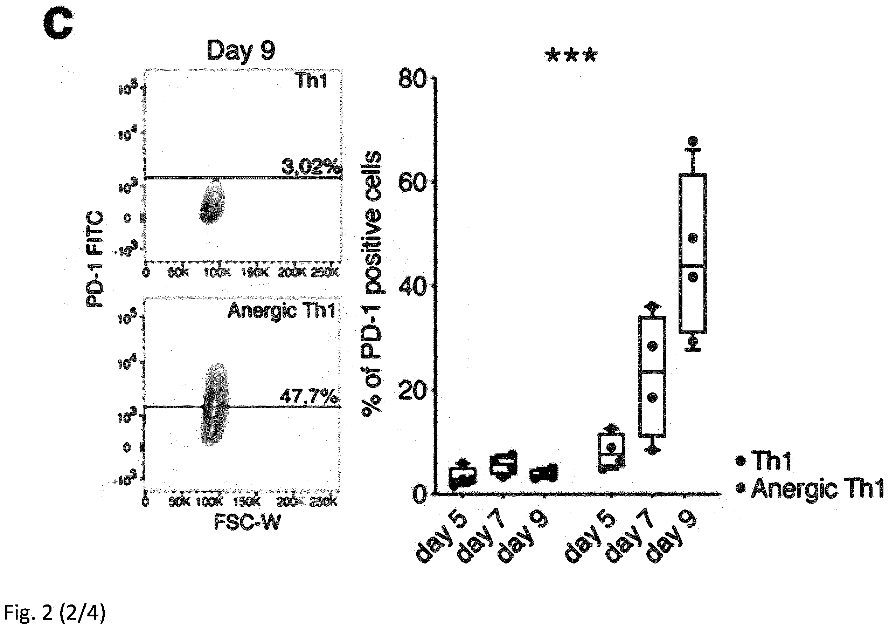

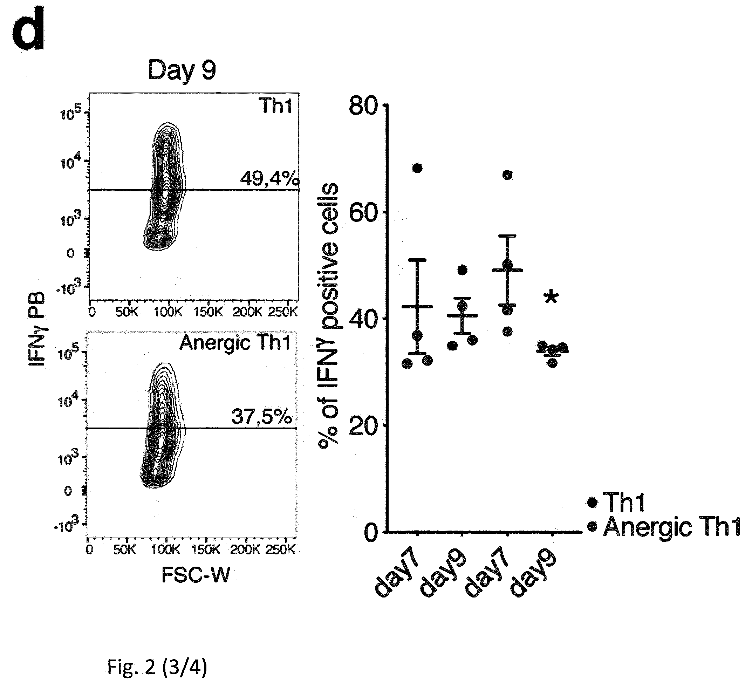

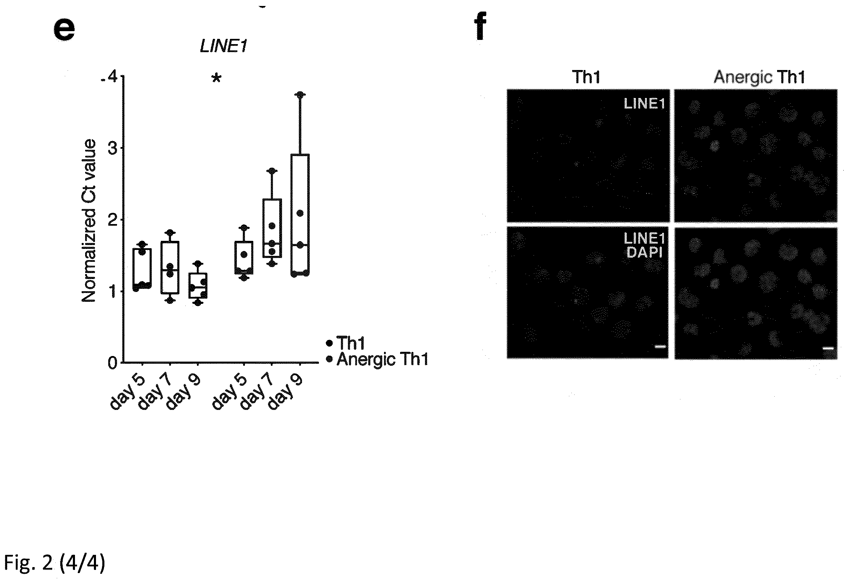

**Figure 2****.** Dysfunctional T cells *in vitro* express chromatin L1 RNAs. a) scheme of the chronical stimulation protocol to obtain anergic Th1 cells b) Anergic Th1 strongly reduce their growth and c) overexpress PD1 marker; d) IFN γ secretion is impaired in Anergic Th1. e) and f) Anergic Th1 homogenously express L1 RNAs on chromatin as revealed by RT-qPCR and RNA-FISH experiments.

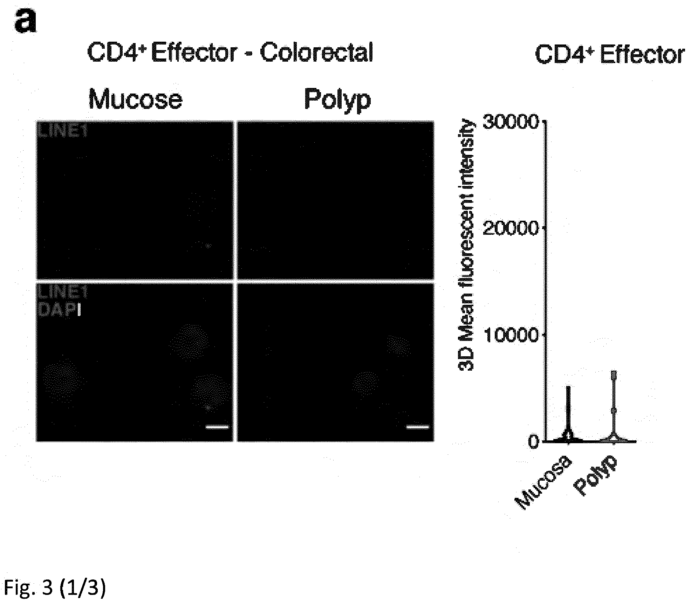

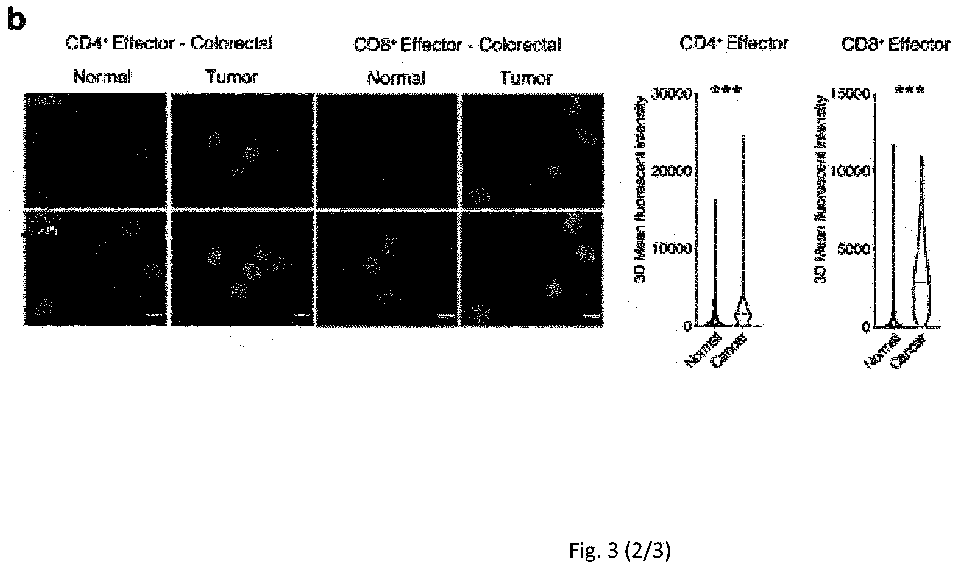

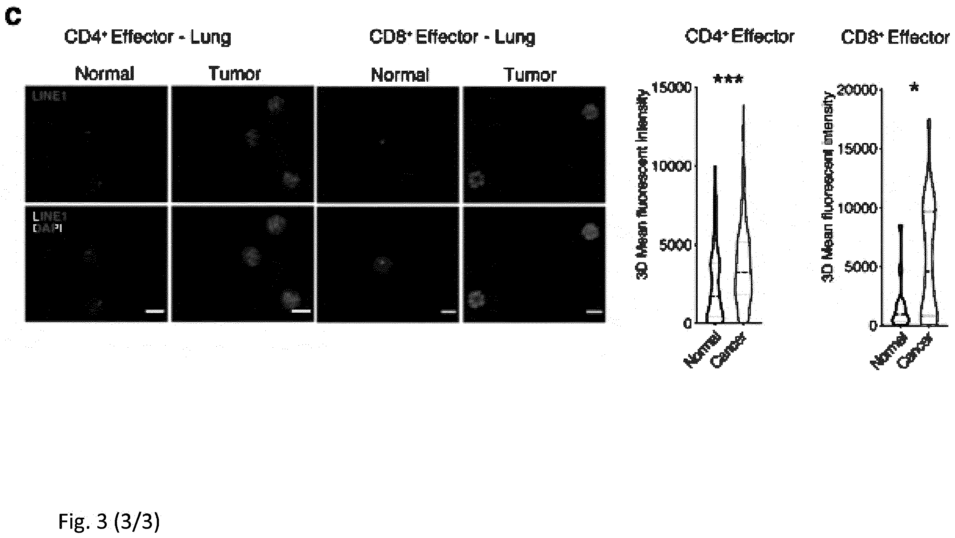

**Figure 3****.** Characterization of L1 RNAs expression in TILs. L1 RNAs are aberrantly expressed in CD4+ and CD8+ Effector T cells isolated from colorectal cancer (a) or lung cancer (b) and not in the normal counterpart, neither in precancerous lesion as polyp, as probed by RNA-FISH experiments. Inventors analysed two different CRC and four different NSCLC patients.

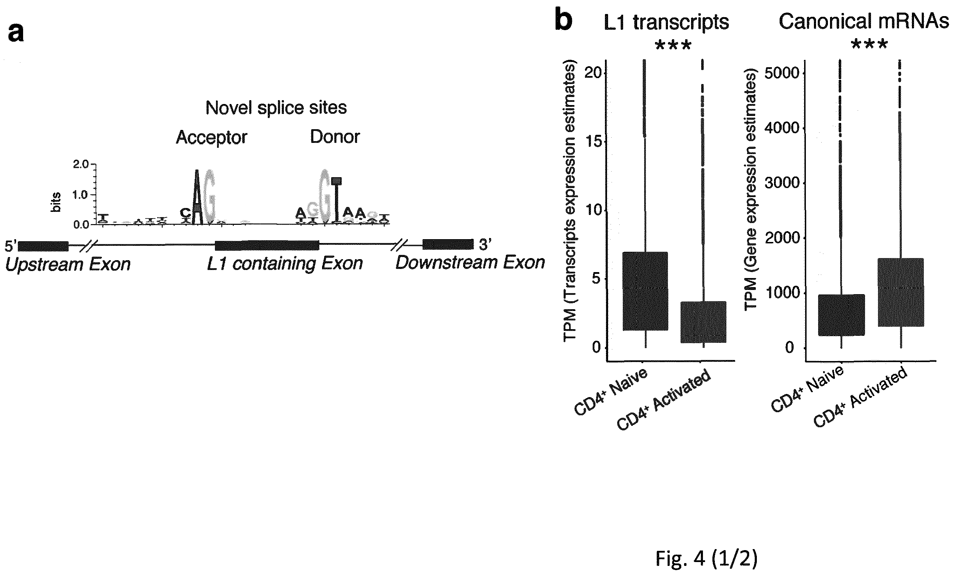

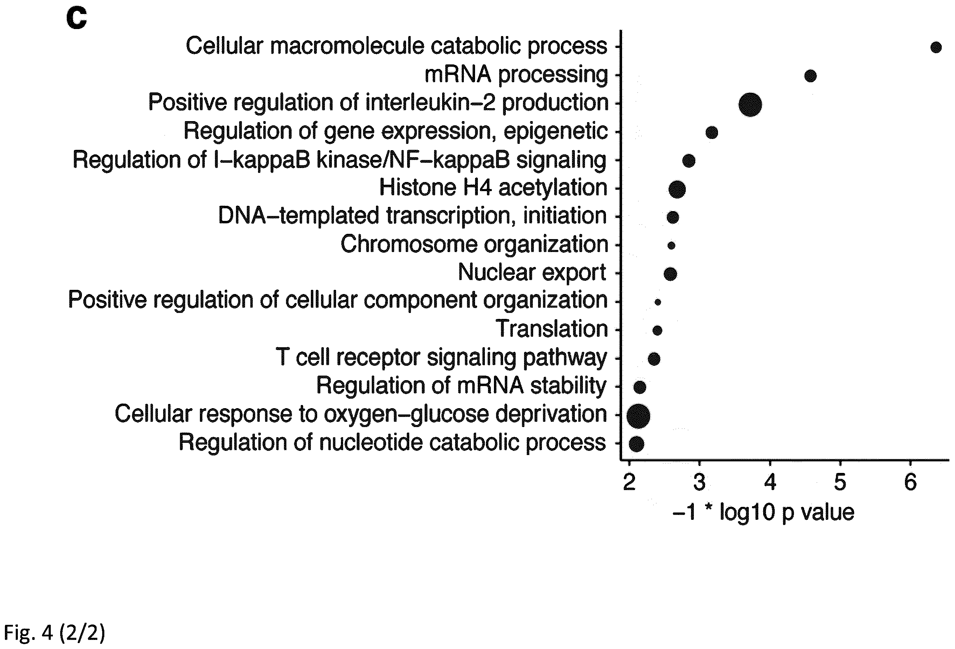

**Figure 4****.** Characterization of novel L1 containing transcripts in human CD4+ naive T cells. a) L1 regions are included in novel exons originating from an intronic portion of protein coding genes;
these novel exons display canonical splicing sites. b) L1 transcripts are downregulated upon T cell activations, whereas the canonical mRNAs of the L1 host genes are upregulated. c) L1 host genes are involved in processes important for T cell activation and function.

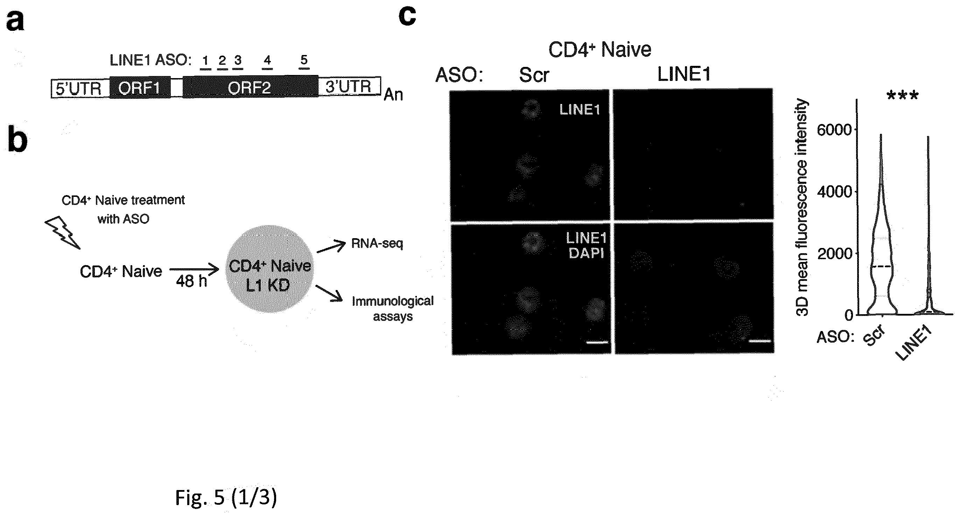

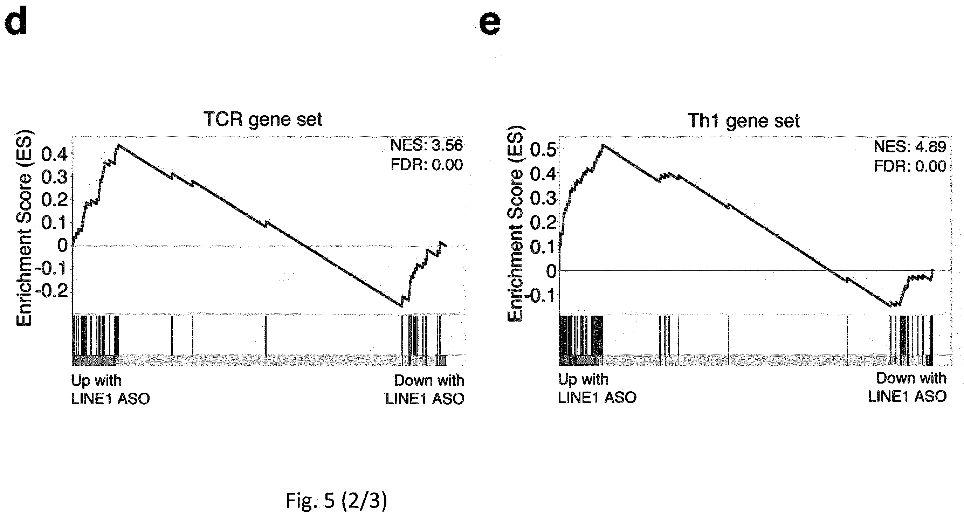

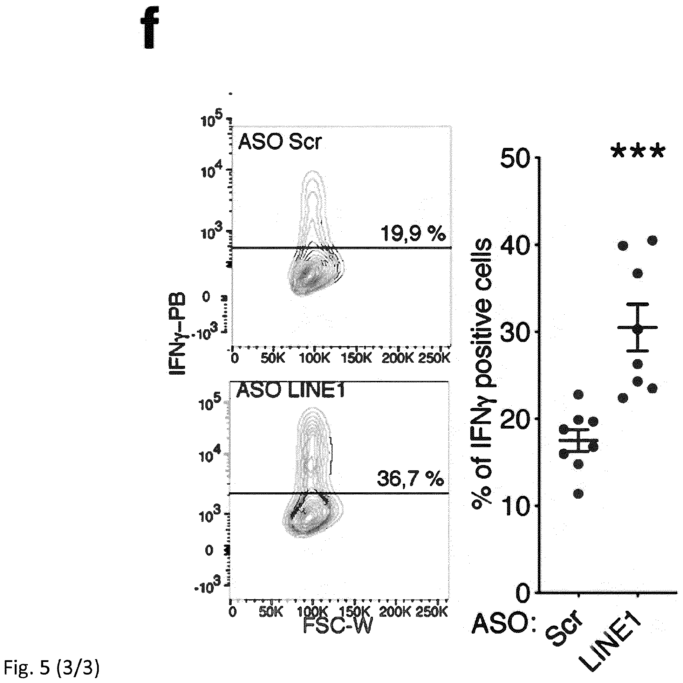

**Figure 5****.** L1 RNAs knock down enhances the effector functions a) and b) schemes for L1 ASO design and knock down experiments. c) Efficiency of L1 RNA knock down measured by RNA-FISH. d and e) GSEA panels indicating that T cells depleted for L1 are transcriptionally enriched for TCR activated genes and Th1 differentiation. f) Differentiating T cells double the secretion of IFNγ upon L1 knock down.

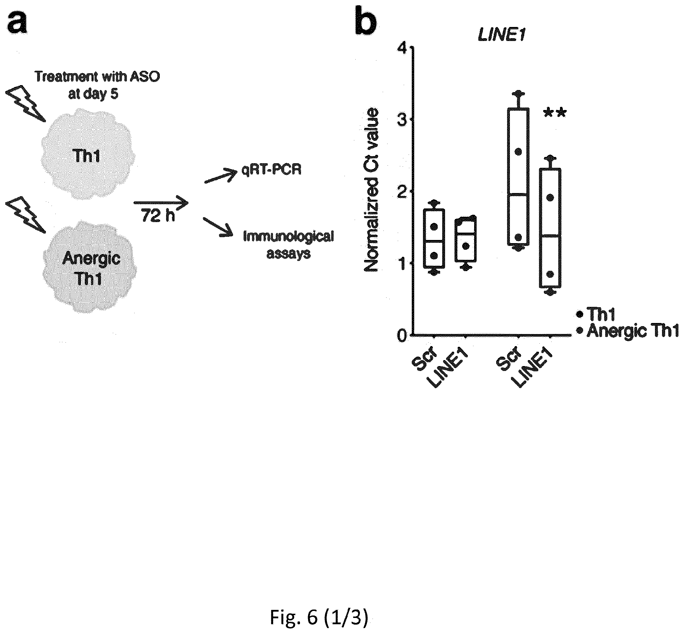

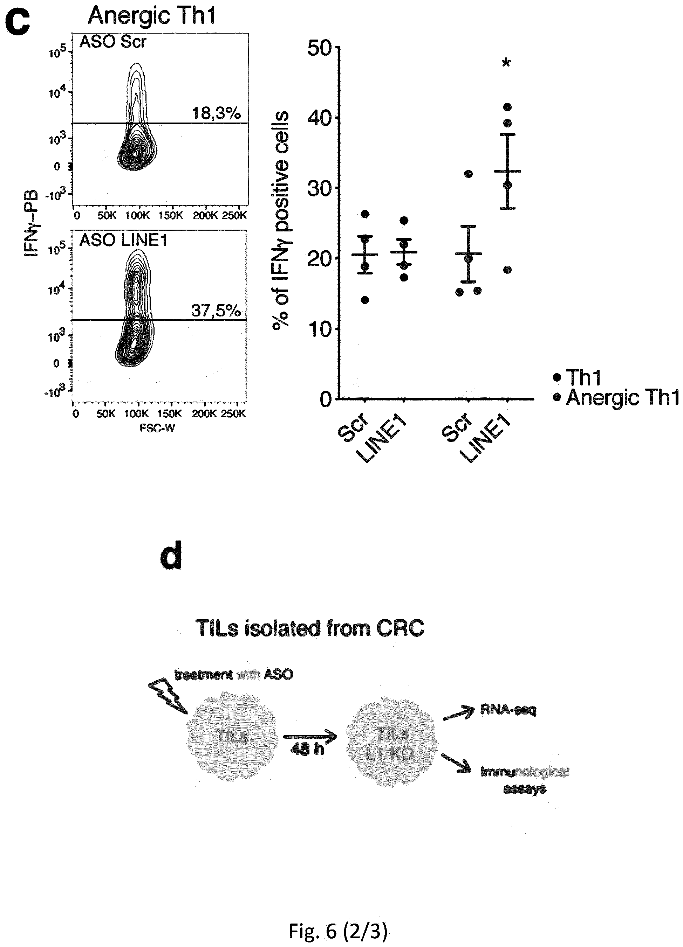

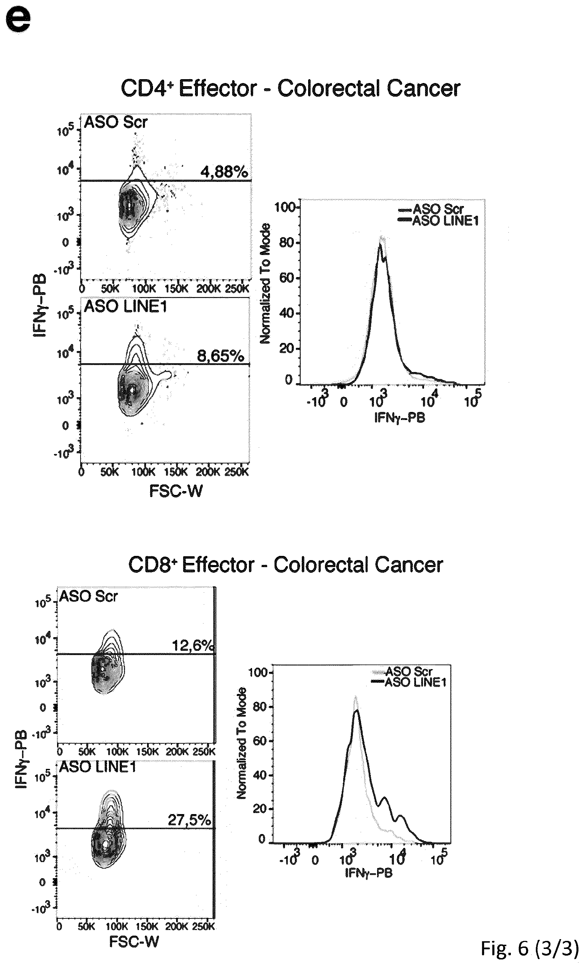

**Figure 6****.** L1 RNAs knock down enhances the effector functions both in dysfunctional T cells *in vitro* and in TILs *ex vivo.* a) and d) schemes for L1 knock down experiments *in vitro* and in TILs *ex vivo.* b) Efficiency of L1 RNAs knock down in dysfunctional T cells *in vitro* measured by RT qPCR. c) Dysfunctional T cells *in vitro* double the secretion of IFNγ upon L1 knock down. e) TILs *ex vivo* isolated from CRC tumor double the secretion of IFNγ upon L1 knock down.

L1 RNAs knock down enhances the effector functions both in dysfunctional T cells *in vitro* and in TILs *ex vivo.* a) and d) schemes for L1 knock down experiments *in vitro* and in TILs *ex vivo.* b) Efficiency of L1 RNAs knock down in dysfunctional T cells *in vitro* measured by RT qPCR. c) Dysfunctional T cells *in vitro* double the secretion of IFNγ upon L1 knock down. e) TILs *ex vivo* isolated from CRC tumor double the secretion of IFNγ upon L1 knock down.

### EXAMPLE 1

### Materials and Methods

### Human blood samples and patients

Buffy coat blood from anonymous healthy donors was provided by Fondazione Istituto di Ricovero e Cura a Carattere Scientifico (IRCCS) Cà Granda Ospedale Maggiore Policlinico in Milan. The age and the sex of healthy donors were unknown for privacy reasons. The ethics committee of Fondazione Istituto di Ricovero e Cura a Carattere Scientifico (IRCCS) Cà Granda Ospedale Maggiore Policlinico approved the use of human blood samples for research purposes and informed consent was obtained from all subjects.

Colorectal cancer (CRC) and Lung cancer samples were obtained from Ospedale San Gerardo Azienda Socio Sanitaria Territoriale (ASST) in Monza. The tumor samples and the normal adjacent tissue were separated from the rest of the tissue by an anatomopathologist and directly transferred to our laboratory. The ethics committee approved the use of tissue samples of cancer patients for research purpose and informed consent was obtained from patients.

### Human primary T cells purification and sorting

Human peripheral blood mononuclear cells (PBMCs) were purified from human blood samples by density gradient centrifugation with Ficoll-Paque Plus, following manufacturer's instructions. From PBMCs, T cells were negatively selected with magnetic separator (AutoMACS Pro Separator; Miltenyi Biotech) using Pan T cell Isolation Kit (Miltenyi Biotec) or CD4+ T cell Isolation Kit (Miltenyi Biotec) following manufacturer's instructions. The isolated CD3+ or CD4+ T cells were stained with the appropriate antibodies for surface markers; T cell subsets were purified by FACS sorting (FACSAria, BD Bioscience) based on surface marker expression. For peripheral blood, PBMCs were directly stained and sorted. CD8+ Naive T cells were sorted as CD8+CD45RO- and CD8+ effector T cells as CD8+CD45RO. CD4+ Naive T cells were sorted as CD4+CD25-CD127-/highCD45RO- and CD4+ Th1 cells as CD4+CD25-CD127-/highCD45RO+CXCR3+, Th2 cells as CD4+CD25-CD127-/highCD45RO+CRTH2+ and Th17 cells as CD4+CD25-CD127-/highCD45RO+CCR6+CXCR3. The purity of sorted cells was >97%.

### Tumor infiltrating lymphocytes isolation and sorting

Tumor and normal tissue were washed several times and maintained overnight at 4°C in Roswell Park Memorial Institute (RPMI) 1640 supplemented with 400 µg/mL gentamicin, 15 µg/mL amphotericin, 500 U/mL penicillin and 500 µg/mL streptomycin. Tissues were weighted, then the samples were smashed and treated for 20 min at 37°C with an EDTA chelation buffer (5 mM EDTA, 1 mM DTT and 67 µg/mL DNase I in HBSS, 5mL per gr of tissue). Tissues were then centrifuged at 550 g at room temperature (RT), were washed with Hank's Balanced Salt Solution (HBSS) (Gibco by Life Technologies) and digested with 1 mg/mL of Collagenase D and 67 µg/mL DNase diluted in HBSS, supplemented with antibiotics; 5mL of digestion solution per gr of tissue were used, tissues were digested at 37°C, 3h, in thermo mixer with max agitation. The digested tissues were filtered with 70 µM cell strainer and washed two times with HBSS for 10 min, 500 g, 4°C. Lymphocytes were stratified through Percoll gradient (100% - 60% - 40% - 30%) for 30 minutes at 400 g, were recovered from the interface between 60% and 40% Percoll layers and were stained with the appropriate antibodies for surface markers; T cell subsets were purified by FACS sorting (FACSAria, BD Bioscience) based on surface marker expression. CD8⁺ effector T cells were sorted as CD45⁺CD3⁺CD8⁺CD45RO⁺. CD4⁺ T regulatory (Treg) cells were sorted as CD45⁺CD3⁺CD4⁺CD25⁺CD127^{low/-}, while from not-Treg cells were sorted CD4⁺ effector T cells as CD45⁺CD3⁺CD4⁺CD45RO⁺.

The following antibodies were used for flow cytometry-based sorting: anti-CD4-APCCy7 (BD Bioscience; clone: RPA-T4); anti-CD8-VioGreen (Miltenyi; clone: REA-734); anti-CD25-PECy7 (Invitrogen by Life Technologies; clone: BC96); anti-CD127-PECy5 (BioLegend; clone: A019D5); anti-CD45RO-BV567 (BioLegend); anti-CD3-PE (BD Bioscence); anti-CD45-Pacific Blue (BD Bioscence).

### CD4⁺ Naive treatment with Actinomycin D

CD4+ Naive T cells were treated in complete RPMI supplemented with 20 IU/mL recombinant IL-2 with Actinomycin D 10 µg/mL (Sigma Cat#A9415) for 16h⁶⁹. Cells will be subjected to qRT-PCR analysis, a spike in RNA had been added to perform normalization in the analysis step.

### In vitro CD4⁺ Naive differentiation to Th1 phenotype

T cells (1,5 x10⁶/mL) were cultured in RPMI 1640 W/GlutaMAX-I medium supplemented with 10% (v/v) FBS, 1% (v/v) non-essential amino acids, ImM sodium pyruvate, 50 U/mL penicillin, 50 µg/mL streptomycin (all from Gibco by Life Technologies), 20 IU/mL recombinant IL-2 (cat. num. 130-097-744), 10 ng/mL recombinant IL-12 (cat. num. 130-0976-704), 2 µg/mL neutralizing anti-IL-4 (cat. num. 130-095-753) (all from Miltenyi) to obtain Th1 cells. Naive CD4⁺ T cells were stimulated with Dynabeads Human T-activator anti-CD3/anti-CD28 beads (1 bead/cell; Gibco by Life Technologies; cat. num. 1131D) and then cultured for at least 9 days.

### In vitro model of CD4⁺ dysfunctional T cells

Naive CD4⁺ T cells were *in vitro* differentiated as described above through Th1 phenotype. Starting from day2 every two days Th1 cells were count and chronically stimulated adding additional Dynabeads Human T-activator anti-CD3/anti-CD28 beads (1 bead/cell; Gibco by Life Technologies), cells will be collected at day 7 and day 9 for subsequent qRT-PCR analysis and immunological staining (cell cycle, transcription factors and cytokines production by intracellular staining (described below)).

### LINE1 knock down in CD4+ Naive, CD4⁺ dysfunctional T cells or tumor infiltrating T cell

For LINE1 RNA knock down experiments was used FANA (2'-deoxy-2'-fluoro-β-D-arabinonucleid acid) - ASOs (antisense oligonucleotides) (AUMbiotech). Five specific FANA ASOs were designed based on LINE1 RNA consensus sequence (Fig. 5a) in order to be able to hit multiple subfamilies. Scramble FANA ASO was used as control. FANA ASOs were added gently pipetting directly in cell medium, they will be delivered by gymnosis without any transfection reagents. Naive CD4⁺ T cells were cultured for 48h in complete medium supplemented with 200 IU/mL recombinant IL-2 (Miltenyi) and FANA-ASOs 10 µM prior being collected for subsequent analysis or differentiated to Th1 phenotype. *In vitro* differentiating Th1 are cultured as described above and maintained with FANA-ASOs 10 µM. CD4⁺ dysfunctional T cells were treated at day 5 with FANA ASO 10 µM and collected for subsequent analysis at day 7 and day 9. Tumor Infiltrating CD4+ and CD8 effector T cells were cultured for 48h in complete medium supplemented with 200 IU/mL recombinant IL-2 (Miltenyi) and FANA-ASOs 10 µM.

Naive CD4⁺ cells knocked down for LINE1 were checked for the absence of activation (CD25/CD9 staining), for their vitality and resting state (cell cycle) and then differentiated toward Th1phenotype. Differentiating ability were assessed with intracellular staining for lineage specific citokines and transcription factors. Dysfunctional T cells knocked down for LINE1 were assessed for their effector abilities with intracellular staining for lineage specific citokines and transcription factors. TILs knocked down for LINE1 were either activated for 48h with CD3/CD28 beads and tested for their effector abilities with intracellular staining for Effector specific citokines. LINE1 RNA knock down efficiency was controlled by RT-qPCR and RNA FISH (described below).

### Surface marker staining, intracellular staining for transcription factors and cytokines production analysis by Flow Cytometry

Surface markers' expression was evaluated by staining T cells for 30 minutes at 37°C in incubator using 1µL of antibody for 5x10⁴ cells. T cells were washed in phosphate-buffered saline (PBS) and then analyzed using FACSCanto I (BD Bioscience). The following antibody was used to stained T cells: anti-CD279 (PD-1)-Alexa Fluor 488 (BioLegend; clone: EH12.2H7).

For intracellular cytokines and transcription factors staining, 5x10⁴ T cells were stimulated at 37°C in incubator for 4h with 50 ng/mL phorbol 12-myristate 13-acetate (PMA) (Merck) and 0,5 µg/mL ionomycin (Merck) in the presence of 100 µg/mL Brefeldin A (Merck) for the last 2 hours. The cells were then fixed and permeabilized for 30 minutes at 4°C with Fixation/Permeabilization solution (Invitrogen by Life Techinologies) according to the manufacturer's instructions. Cytokines and transcription factor were so stained using for 5x10⁴ cells 1µL of antibody diluited in Permabilization Buffer (IX) (Invitrogen by Life Techinologies) for 20 minutes at room temperature. T cells were washed in PBS and then analyzed with FACSCanto I (BD Bioscience). For the intracellular staining was used the following antibody: anti-IFN-γ-V450 (clone: B27),. An average of 10⁴ cells were acquired per sample for all type of staining and data were analyzed using FlowJo v.10 software.

### RNA extraction, cDNA synthesis, and real-time quantitative RT-PCR

Total RNA was isolated using RNeasy Mini Kit (QIAGEN) plus QIAshredder (QIAGEN) according to manufacturer's instruction. During the extraction a step of DNAse with RNase-free DNase Set (QIAGEN) for 40 minutes at 30°C was performed to remove any residual DNA. The eluted RNA was quantified by NanoDrop (Thermo Scientific by Life Technologies) and then cDNA was synthesized using SuperScript III First-Strand Synthesis SuperMix kit (Invitrogen by Life Technologies) following manufacturer's instructions. In brief, a step of reverse transcription was followed by a step of RNA hydrolyzation with *E. coli* RNase H (30 min at 37°C). Real-time quantitative PCR was performed on StepOnePlus Real-Time PCR System (Applied Biosystem by Life Technologies), using Power SYBR Green PCR Master mix (Applied Biosystem by Life Technologies). Transcript expression levels were normalized on housekeeping gene and reported as normalized value.

### 3D RNA-FISH and 3D fluorescent intensity analysis

To perform 3D RNA-FISH an RNA biotinylated probe was produced by *in vitro* transcription of PCR amplicons, followed by RNAse digestion and precipitation. With this protocol was generated RNA probes to detect LINE1 using a consensus sequence. To perform 3D RNA-FISH 2x10⁵ T cells were seed on poly-lisinated 10 mm glasses for 30 minutes and were fixed with 3% parafolrmaldehyde (PFA) for 10 min. Then the cells were washed three times for 5 min with 0,05% Triton-X-100 in PBS, were permeabilized with 0,5% Triton-X-100 in PBS for 10 minutes at room temperature and were maintained for an overnight in 20% glycerol in PBS at 4°C. Glasses were then freezed and thawed on dry ice followed by soaking with 20% glycerol in PBS, T cells were washed 5 min with 0,5% Triton-X-100 in PBS and two times for 5 min with ,05% Triton-X-100 in PBS, and were incubated for 15 min with 0.1 M HCl. Then T cells were rinsed in 2X SSC and hybridized with RNA probe (4 µL/glass) at 52.5°C for 3.30 minutes followed by overnight at 37°C in a humid chamber. Hybridization was followed by a detection step in which glasses were washed three times (5 min, 39°C) with 50% formamide in 2X SSC, three times (5 min, 39°C) with 2X SSC, one time (5 min, RT) on an orbital shaker with IX SSC, two times (5 min, RT, orbital shaker) with 4X SSC, 0,2% Tween-20 in DEPC. T cells were blocked in TBN/ BSA (0.1 M TrisHCl pH 8, 0.150 M NaCl, 4% BSA in DEPC) for 30 min at RT on an orbital shaker and then incubated (30 min, RT, orbital shaker) with Streptavidin HRP (1:500; Perkin Elmer by Akoya Biosciences) diluted in TNT/BSA (0.1 M TrisHCl pH 8, 0.150 M NaCl, 0,1% NP-40, 4% BSA in DEPC). T cells were washed 4 times (5 min, orbital shaker) with TNT and then a signal amplification was performed with TSA Plus Cyanine 3.5 (Perkin Elmer by Akoya Biosciences) incubating T cells with TSA working solution (1:100) in IX amplification buffer for 3.5 minutes at room temperature, mild shaking. T cells were washed 4 times (5 min, orbital shaker) with TNT and post-fixed in 2% formaldehyde in PBS for 2 min at room temperature. After brief washes in PBS, nuclei were counterstained with 1 µg/mL DAPI (4,6-diamidino-2-phenylindole) in PBS for 5 minutes at room temperature. To quantified 3D mean fluorescence intensity of RNA signal, images were analyzed with NIS-Elements Software (by Nikon): with command "General Analysis" a mask on DAPI signals was generated to identify a single nuclei, and then was performed a "3D measurement" of RNA signals in every nuclei identify. While to measure the colocalization of RNA and histone mark signals was used ImageJ Software to control the Pearson Correlation through the command "Colocalization Threshold" for every nucleus.

### Chromatin RNA extraction and library preparation for chromatin RNAseq

Cellular fractionation was performed as published in ⁷⁰ with minor modifications. In brief, at least 3-10x10⁶ T cells were pelleted (500 x g, 10 min, 4°C) and resuspended ten times gently in 30 µL of Buffer A (10 mM HEPES pH 7.5, 10 mM KCl, 10% (v/v) glycerol, 340 mM sucrose, 4 mM MgCl₂, 1 mM DTT, IX Protease Inhibitor Cocktail (PIC)), then was added to T cells an equal volume of Buffer A plus 0,2% (v/v) Triton X-100, always T cells were resuspended ten times gently. To lyse T cells the solution was incubated for 12 minutes on ice and then was centrifugated at 1200 x g for 5 minutes at 4°C to recover cytoplasmic fraction. The nuclear pellet was washed (900 x g, 5 min, 4°C) in NRB Buffer (20 mM HEPES pH 7.5, 50 % (v/v) glycerol, 75 mM NaCl, 1 mM DTT, IX PIC) and then resuspended in 30 µL of NRB Buffer plus equal volume of NUN Buffer (20 mM HEPES pH 7.5, 300 mM NaCl, 1 M Urea, 1 % (v/v) NP-40, 1 mM MgCl₂, 1 mM DTT). The solution was incubated for 5 minutes on ice, and then the nuclear fraction was collected after a centrifuge (1200 x g, 5 min, 4°C). The residual pellet was washed in 500 µL of Buffer A (1200 x g, 5 min, 4°C) and then the pellet was collected as chromatin fraction in 50 µL of Buffer A. In every fraction collected was added 200 µL of Homogenization Buffer and then stored at - 80°C prior to use. The RNA has been extracted with Maxwell Promega Technology (Promega. Cod. AS 1460). 15-75ng of chromatin associated RNA has been used for library preparation using SMARTer Stranded RNA-Seq Kit (Takara cod 634861). Libraries have been sequenced as paired, 150 pb of leght on Illumina NextGen 500.

### De novo reconstruction of L1 containing transcripts

Fastq files will be checked for read quality with FastQC, adapters will be removed, rRNA reads will be filtered against human ribosomal RNA sequences and trimming will be performed with BBDuk⁷¹. Quality passed read pairs will be used for alignment using STAR⁷² against human genome (hg38). Properly paired reads will be selected using Samtools suite⁷³. For *de novo* transcriptome assembly, two independent assemblers will be used: Trinity⁷⁴ in genome guided in tandem with PASA⁷⁵, and StringTie⁷⁶. Mono-exonic transcripts will be removed and multi-exonic transcripts intersecting with any transposable element (UCSC Repeatmasker) will be selected. Transcripts sharing the TEs containing exon identified by both the assemblers⁷⁷ will be used to make a unified TEs containing transcriptome. Transcripts with at least one exon intersecting with L1 will be identified as L1 *de novo* transcripts. TE transcripts will be annotated using gffcomapre against a reference transcript GTF file composed of GENCODE and non-coding RNAs identified in CABILI⁷⁸, FANTOM 5⁷⁹ and other groups^{80,81}. To retrieve consistent L1 novel transcripts, inventors will consider only those common between ≥ 3 out of 5 biological replicates.

### Results

Inventors herein provide observations regarding the dynamics of LINE1 expression in human T lymphocytes from peripheral blood, in dysfunctional/anergic T cells *in vitro* and in TILs, and their identification in CD4+ naive T cells. Inventors probed TEs expression in RNA FISH and RT-qPCRs experiments, finding that SINEs are broadly expressed, LTRs are repressed, while LINEs (LINE1 in particular) are specifically enriched in CD4+ T naive nuclei and not in CD4+ T effectors or CD8+ T cells; L1 RNAs are chromatin bound, localized in euchromatin regions (H3K4me3, H3K36me3) avoiding heterochromatin (H3K9me3), foreseeing a ncRNAs regulatory function on active genes. Upon Naive activation, L1 RNAs are rapidly downregulated (Fig. 1). Further, inventors have set up a protocol of chronically stimulation of T effector cells *in vitro* that render the cells anergic (cell cycle arrest, PD1 overexpression and less IFN γ production); in such conditions, T cells are strongly enriched in chromatin L1 RNAs (Fig. 2). Inventors have also analyzed L1 expression in TILs, finding that CD4+ and CD8+ T effectors within the tumor aberrantly express L1 RNAs at chromatin, in respect to T cells infiltrating the normal tissue. In colorectal cancer, this feature is exclusively tumor specific, as T lymphocytes infiltrating normal mucosa and precancerous polyps from the same patients do not express L1 RNAs. Inventors observed similar trends both for CRC and NSCLC tumors and for different patients, suggesting that this is a common and specific feature to the TILs (Fig.3).

To identify the L1 transcripts expressed in CD4+ naive T cells, inventors set up a strategy of deep sequencing of chromatin associated RNAs (to enrich for L1 RNAs molecules) coupled with a dedicated pipeline of *the novo* reconstruction of L1 containing transcripts. Interestingly, inventors found that the majority of expressed L1 RNAs (94%) belong to evolutionary old L1 subfamilies (L1M), short in length (380 bp on average) and therefore inactive (unable to retrotranspose⁶⁶); they are mainly comprised in protein coding genes (93%), in the introns (72%). These specific L1 regions are included in novel exons, originating from an intronic portion of the gene that contain the L1 sequence and display donor/acceptor canonical splicing sites (Fig. 4a). Interestingly, inventors found that these transcripts are chromatin associated alternative isoforms, that are expressed in resting CD4+ naive cells and downregulated upon T cell activation; while the canonical mRNAs of the L1 host genes are upregulated (Fig. 4b). The genes regulated by this mechanism are involved in processes important for immune response (Fig. 4c). Inventors are suggesting that the inclusion of L1 novel exons could interfere with the transcription of the canonical protein coding mRNA of such genes, as previously observed^{67,68}.

Inventors have already efficiently set up L1 RNAs knock down in CD4+ T naive cells (resting cells) without activating them or altering the cell cycle or inducing apoptosis. The strategy takes advantage of the use of modified antisense oligonucleotides (FANA ASO) designed on a consensus sequence of L1; as exemplified in Fig. 5c, this assay could knock down most of the L1 signal. Interestingly, L1 depleted CD4+ naive cells upregulate at transcriptional levels genes involved in T cell activation and differentiation (Fig. 5d and e), and they double the amount of effector cytokines secretion after 7 days of differentiation (Fig. 5f).

As a pivotal experiment, inventors used the same L1 ASOs cocktails to knock down L1 RNAs in dysfunctional T cells *in vitro* and TILs *ex vivo;* inventors obtained that L1 knock down increases IFN γ secretion in TILs, suggesting a recovery of their dysfunctional state. Inventors demonstrated that also in this context, L1 knock down is able to increase the effector functions. (Fig. 6).

### Sequences

Consensus sequence of LINE1 used to design the oligonucleotides a-b-c-d-e, i.e. SEQ ID Nos:2-6 (in grey the regions of greatest homology among the LINE1 families that were used to generate the consensus; dashes correspond to n (i.e. a or g or c or t nucleotide))

g-----------------------------------------------------------
------------------------------------------------------------
------------------------------------------------------------
------------------------------------------------------------
------------------------------------------------------------
-------ggggaggagccaagatggccgaataggaacagctccggtctacagctcccagc
gtgagcgacgcagaagacgg-tgat-ttctgcatttccatctgaggtaccgggttcatct
cactagggagtgccagacagtgggcgcaggccagtgtgtgtgcgcaccgtgcgcgagccg
aagcagggcgaggcattgcctcacctggg---aagcgcaaggggtcagggagttcccttt
ctgagtcaaagaaaggggtgacggtcg-cacctggaaaatcgggtcactcccacccgaat
attgcgcttttcagaccggcttaagaaacggcgcaccacgagactatatcccacacctgg
c--tcggagggtcctacgcccacggaatctcgctgattgctagcacagcagtctgagatc
aaactgcaaggcggcaacgaggctgggggaggggcgcccgccattgcccaggcttgctta
ggtaaacaaagcagccggg-aagctcgaactgggtggagcccaccacagctcaaggaggc
ctgcctgcctctgtaggctccacctctgggggcagggcacagacaaacaaaaaggcagca

gtaacctctgcagacttaagtgtccctgt-ctgacagctttgaagagagcagtggttctc
ccagcacgcagctggagatctgagaacgggcagacagactgcctcctcaagtgggtccct
gactcctgacccccgagcagcctaactgggaggcacccccagcaggggcacactgacac
ctcaca-cggcagggtattccaacagacctgcagctgagggtcctgtctgttagaaggaa
aactaacaaccag--aaaggacatctacaccgaaacccatctgtacatcaccatcatca
aagaccaaaagtagataaaccacaaagatgggaaaaaacagaacagaaaaactggaaa
ctctaaaacgcagagcgcc-tctcctcctccaaaggaacgcagttcctcaccagcaacag
aacaaagctggatggagaatg-atttgacgagctgagagaagaaggcttcagacgat--
-------caaattactctgagctacgggaggacattcaaaccaaaggcaaagaagttgaa
aactttgaaaaaaatttagaagaatgtataactagaataaccaatacagagaagtgctta
aaggagctgatggagctgaaaaccaaggctcgagaactacgtgaagaatgcagaagcctc
aggagccgatgcgatcaactggaagaaagggtatcagcaatggaagatgaaatgaatgaa
atgaagcga-gaagggaagtttagagaaaaagaataaaaagaaatgagcaaagcctcca
agaaatatgggactatgtgaaaagaccaaatctacgtctgattggtgtacctgaaagtga
tgtggagaatggaaccaagttggaaaacactctgcaggatattatccaggagaacttccc
caatctagcaaggcaggccaacgttcagattcaggaaatacagagaacgccacaaagata
ctcctcgagaagagcaactccaagacacataattgtcagattcaccaaagttgaaatgaa
ggaaaaaatgttaagggcagccagagagaaaggtcgggttaccctcaaaggaaagcccat
cagactaacagtggatctctcggcagaaaccctacaagccagaagagagtgggggccaat
attcaacattcttaaagaaaagaatttcaacccagaatttcatatccagccaaactaag
cttcataagtgaa---ggagaaataaaatactttatagacaagc-aaatgttgagagatt
ttgtcaccaccaggcctgccctaaa--agagctc-ctgaaggaagcgctaaacatggaaa
ggaacaaccggtaccag-ccgctgcaaaatcatgccaaaatgtaaagaccattgagacta
ggaagaaactgcatcaactaatgagcaaaatcaccagctaacatcataatgacaggatca
aattcacacataacaatattaactttaaatataaatggactaaattctgcaattaaaaga
cacagactggcaagttggataaagagtcaagacccatcagtgtgctgtattcaggaaacc
catctc-acg-tgcagagacacacataggctcaaaataaaaggatggaggaagatctacc
aagccaatggaaaacaaaaaaaggcaggggttgcaatcctagtctctgataaaacagact
ttaaaccaacaaagatcaaaagagacaaagaaggccattacataatggtaaaggatcaa
ttcaacaagaggagctaactatcctaaa----tatttatgcacccaatacaggagcaccc
agattcataaagcaagtcctgagtgacctacaaagagacttagactcccacacattaata
atgggagactttaacacccactgtcaacattagacagatcaacgagacagaaagtcaac
aaggatacccaggaattgaact--cagctctgcaccaagcagaccta-atagacatctac
agaactctccaccccaaatcaacagaatatacctttttt-cagcaccacaccacaccta
ttccaaaattgaccacatagttgga---agtaaagctctcctcagcaaatgtaaaagaac
agaaattataacaaactatctctcagaccacagtgcaatcaaactagaactcaggattaa
gaatctcactcaaagccgctcaactacatggaaactgaacaacctgctcctg-aatgact
actgggtacataacgaaatgaaggcagaaataaagatgttctttg---aaaccaacgaga
acaaagacaccacataccaga---atctctgggacgcattcaaagcagtgtgtagaggga
aatttatagcactaaatgcctacaagagaaagcaggaaagatccaaaattgacaccctaa
catcacaattaaaagaactagaaaagcaagagcaaacacattcaaaagctagcagaa-gg
caagaaataactaaaatcagagcagaactg-aaggaaatagagacacaaaaacccttca
aaaaatcaatgaatccaggagctggtttttgaaaggatcaacaaaattgatagaccgct
agc-aagactaataaagaaaaaagagagaagaatcaaatagacacaataaaaaatgata
aaggggatatcaccaccgatcccacagaaatacaaactaccatcagagaatactacaaac
acctctacgcaa---ataaactagaaaatctagaagaa-atggatacattcctcgacaca
tacactctcccaagactaaaccaggaag-aagttgaatctctgaatagaccaataacagg
ct-ctgaaattgtg---gcaataatcaatagtttaccaaccaaaaagagtccaggaccag
atggattcacag-c---cgaattctaccagaggtacatggaggaactggtaccattcctt
ctgaaactattcc-------aatcaatagaaaaagagggaatcctcc-ctaactcatttt
atgaggccagcatcattctgataccaaa-gccgggcagagacacaaccaaaaa-----ag
agaattt-tagaccaatatccttgatgaa-----c------attgatgcaaaaatcctca
ataaaatactggcaaaccgaatccattagcacatcaaaaagcttatccaccatgatcaag
tgg-gcttcatccctgggatgcaaggctggttcaata--tacgcaaatcaataaatgtaa
tccagcatataaa-cagagccaaagacaaaaaccacatgattat--ctcaatagatgcag
aaaa-agcctttgacaaaattcaacaacccttcatgctaaaaactctcaataa--attag
gtattgatgggacgtatttcaaaataataagagctatctatgacaaacccacagccaata
tcatactgaatgggcaaaaactggaagcat-tccctttgaaaa-ccggcacaagac-agg
gatgccctctctcaccgctcctattc----aacatagtgttg--gaagtt--ctggccag
ggcaatcaggcaggagaaggaaataaagggtattcaattaggaaaagaggaagtcaaatt
gtccctgtttgcagacgacatgattgtatatc-tagaaaaccccatcgtctcagcccaaa

atc-tccttaagctgataagcaacttcagcaaagtctcaggatacaaaatc-aatgtaca
aaaatcacaagcattcttatacaccaacaacagacaaacagagagccaaatcatggg--t
gaac----tcccattcgtaattgc--ttcaaagagaataaaatacctaggaatccaactt
acaagggatgtgaaggacctcttcaaggagaactacaaaccactgctcaaggaaataaaa
gaggacacaaacaaatggaagaacattccatgctcatgggtaggaagaatcaatatcgtg
aaaatggccatactgccca-aggtaatttacagattcaatgccatccccatcaagctacc
aatgactttcttcacagaat----tggaaaaaactactttaaagttcatatggaaccaaa
aaagagcc-cgcattgccaagtcaatcctaagccaaaagaacaaagctggaggcatcaca
ctacctgacttcaaac--tatactacaaggctacagtaaccaaaacagcatgg-tactgg
taccaaaacagagatatagatcaatggaacagaacagagccctcagaaataatgccgcat
atctacaactatctgatctttg-acaaacctgagaaaaacaagcaatggggaaaggattc
cctatttaataaatggtgctgg-gaaaactggctagccatatgtagaaagctgaaactgg
atcccttccttacaccttatacaaaaatcaat----tca----agatggattaaagattt
aaacgttaaacctaaaaccataaaa----accctagaagaaaacctaggcattaccattc
a--ggacataggcgtgggcaaggacttcatgt-----ccaaaacaccaaaagcaatggca
acaaaagacaaaattgacaaatgg---gatctaattaaactaaagagcttctgcacagca
aaagaaactaccatcagagtgaa--caggcaacctacaacatggagaaaatttcgcaa
cctactcatctga-caaaggctaatatccagaatctacaatgaactcaaacaaatttac
aagaaa---aaaacaaacaacccatcaaaaagtgggtgaaggacatgaacagacacttc
tcaaaagaagacatttatgcagccaaa-aaacacatgaagaaatgctcatcatcactggc
catcagagaaatgcaaatcaaaaccactatgagatatcatctcacaccagtt--agaatg
gcaatca-ttaaaaag-tcaggaaacaacaggtgctggagaggatgcgga-gaaatagga
acactttacactgttggtgggactgtaaactagttcaaccattgtggaagtcagtgtgg
c--gattcctcaggatctag-aactagaaataccatttgacccagccatcccattact-
---gggtatatacc-caaatgagtataaatcatgctg--ctataagacacatgcacacg
t--atgtttattgcggcactat----tca------caatagcaaagacttggaaccaacc
ca---aatgtccaac-aatgatagactggattaagaaaatgtggcacatatacaccatgg
a--atactatgcag-----ccataaaaaatgatgagttcatatcctttgtagggacatgg
atgaaattgg-----aaa--ccatcattctcagtaaactatcgcaagaacaaaaaaccaa
acaccgca---tattctcactcataggtgggaattg--aacaa-tgagatcacatggaca
caggaaggggaatatcacactctgg---------ggactgtggtggggtcggg-ggaggg
gggaggatagcattg-----ggagatatacctaatg--ctagatgacacattagtgggt
gcagcgcaccagcatggcacatgtatacatatgtaactaacctgcacaatgtgcacatgt
accctaaaacttagagtataataaaaaaaaaaaaaaaaaaaaaaa--------
------------------------------------------------------------
------------------------------------------------------------
------------------------------------------------------------
------------------------------------------------------------
------------------------------------------------------------
------------------------------------------------------------
------------------------------------------------------------
------------------------------------------------------------
-----------------------a
[SEQ ID NO:1]

### References

1. de Koning, A.P., Gu, W., Castoe, T.A., Batzer, M.A. & Pollock, D.D. Repetitive elements may comprise over two-thirds of the human genome. PLoS Genet 7, e1002384 (2011).
2. Lander, E.S. et al. Initial sequencing and analysis of the human genome. Nature 409, 860-921 (2001).
3. Chuong, E.B., Elde, N.C. & Feschotte, C. Regulatory activities of transposable elements: from conflicts to benefits. Nat Rev Genet 18, 71-86 (2017).
4. Feschotte, C. Transposable elements and the evolution of regulatory networks. Nat Rev Genet 9, 397-405 (2008).
5. Percharde, M., Sultana, T. & Ramalho-Santos, M. What Doesn't Kill You Makes You Stronger: Transposons as Dual Players in Chromatin Regulation and Genomic Variation. Bioessays 42, e1900232 (2020).
6. Kazazian, H.H., Jr. & Moran, J.V. The impact of L1 retrotransposons on the human genome. Nat Genet 19, 19-24 (1998).
7. Viollet, S., Monot, C. & Cristofari, G. L1 retrotransposition: The snap-velcro model and its consequences. Mob Genet Elements 4, e28907 (2014).
8. Okada, N., Hamada, M., Ogiwara, I. & Ohshima, K. SINEs and LINEs share common 3' sequences: a review. Gene 205, 229-43 (1997).
9. Malik, H.S., Henikoff, S. & Eickbush, T.H. Poised for contagion: evolutionary origins of the infectious abilities of invertebrate retroviruses. Genome Res 10, 1307-18 (2000).
10. Canapa, A., Barucca, M., Biscotti, M.A., Forconi, M. & Olmo, E. Transposons, Genome Size, and Evolutionary Insights in Animals. Cytogenet Genome Res 147, 217-39 (2015).
11. Kazazian, H.H., Jr. Mobile elements: drivers of genome evolution. Science 303, 1626-32 (2004).
12. Belancio, V.P., Hedges, D.J. & Deininger, P. Mammalian non-LTR retrotransposons: for better or worse, in sickness and in health. Genome Res 18, 343-58 (2008).
13. Muotri, A.R. et al. Somatic mosaicism in neuronal precursor cells mediated by L1 retrotransposition. Nature 435, 903-10 (2005).
14. Beck, C.R. et al. LINE-1 retrotransposition activity in human genomes. Cell 141, 1159-70 (2010).
15. Ewing, A.D. & Kazazian, H.H., Jr. High-throughput sequencing reveals extensive variation in human-specific L1 content in individual human genomes. Genome Res 20, 1262-70 (2010).
16. Iskow, R.C. et al. Natural mutagenesis of human genomes by endogenous retrotransposons. Cell 141, 1253-61 (2010).
17. Baillie, J.K. et al. Somatic retrotransposition alters the genetic landscape of the human brain. Nature 479, 534-7 (2011).
18. Perrat, P.N. et al. Transposition-driven genomic heterogeneity in the Drosophila brain. Science 340, 91-5 (2013).
19. Upton, K.R. et al. Ubiquitous L1 mosaicism in hippocampal neurons. Cell 161, 228-39 (2015).
20. Coufal, N.G. et al. L1 retrotransposition in human neural progenitor cells. Nature 460, 1127-31 (2009).
21. Reilly, M.T., Faulkner, G.J., Dubnau, J., Ponomarev, I. & Gage, F.H. The role of transposable elements in health and diseases of the central nervous system. J Neurosci 33, 17577-86 (2013).
22. Saleh, A., Macia, A. & Muotri, A.R. Transposable Elements, Inflammation, and Neurological Disease. Front Neurol 10, 894 (2019).
23. Payer, L.M. & Burns, K.H. Transposable elements in human genetic disease. Nat Rev Genet 20, 760-772 (2019).
24. Deniz, O., Frost, J.M. & Branco, M.R. Regulation of transposable elements by DNA modifications. Nat Rev Genet 20, 417-431 (2019).
25. Mc, C.B. The origin and behavior of mutable loci in maize. Proc Natl Acad Sci U S A 36, 344-55 (1950).
26. McClintock, B. Controlling elements and the gene. Cold Spring Harb Symp Quant Biol 21, 197-216 (1956).
27. Sundaram, V. et al. Widespread contribution of transposable elements to the innovation of gene regulatory networks. Genome Res 24, 1963-76 (2014).
28. Bodega, B. & Orlando, V. Repetitive elements dynamics in cell identity programming, maintenance and disease. Curr Opin Cell Biol 31, 67-73 (2014).
29. Pennisi, E. Genomics. ENCODE project writes eulogy for junk DNA. Science 337, 1159, 1161 (2012).
30. Bourque, G. et al. Evolution of the mammalian transcription factor binding repertoire via transposable elements. Genome Res 18, 1752-62 (2008).
31. Imbeault, M., Helleboid, P.Y. & Trono, D. KRAB zinc-finger proteins contribute to the evolution of gene regulatory networks. Nature 543, 550-554 (2017).
32. Morgan, H.D., Sutherland, H.G., Martin, D.I. & Whitelaw, E. Epigenetic inheritance at the agouti locus in the mouse. Nat Genet 23, 314-8 (1999).
33. Ferrari, R. et al. TFIIIC Binding to Alu Elements Controls Gene Expression via Chromatin Looping and Histone Acetylation. Mol Cell 77, 475-487 e11 (2020).
34. Schmidt, D. et al. Waves of retrotransposon expansion remodel genome organization and CTCF binding in multiple mammalian lineages. Cell 148, 335-48 (2012).
35. Zhang, Y. et al. Transcriptionally active HERV-H retrotransposons demarcate topologically associating domains in human pluripotent stem cells. Nat Genet 51, 1380-1388 (2019).
36. Faulkner, G.J. et al. The regulated retrotransposon transcriptome of mammalian cells. Nat Genet 41, 563-71 (2009).
37. Rodriguez-Terrones, D. et al. A distinct metabolic state arises during the emergence of 2-cell-like cells. EMBO Rep 21, e48354 (2020).
38. Lu, J.Y. et al. Genomic Repeats Categorize Genes with Distinct Functions for Orchestrated Regulation. Cell Rep 30, 3296-3311 e5 (2020).
39. Attig, J. et al. Heteromeric RNP Assembly at LINEs Controls Lineage-Specific RNA Processing. Cell 174, 1067-1081 e17 (2018).
40. Nekrutenko, A. & Li, W.H. Transposable elements are found in a large number of human protein-coding genes. Trends Genet 17, 619-21 (2001).
41. Perepelitsa-Belancio, V. & Deininger, P. RNA truncation by premature polyadenylation attenuates human mobile element activity. Nat Genet 35, 363-6 (2003).
42. Roy-Engel, A.M. et al. Human retroelements may introduce intragenic polyadenylation signals. Cytogenet Genome Res 110, 365-71 (2005).
43. Gong, C. & Maquat, L.E. IncRNAs transactivate STAU1-mediated mRNA decay by duplexing with 3' UTRs via Alu elements. Nature 470, 284-8 (2011).
44. Kapusta, A. et al. Transposable elements are major contributors to the origin, diversification, and regulation of vertebrate long noncoding RNAs. PLoS Genet 9, e1003470 (2013).
45. Kelley, D. & Rinn, J. Transposable elements reveal a stem cell-specific class of long noncoding RNAs. Genome Biol 13, R107 (2012).
46. Fort, A. et al. Deep transcriptome profiling of mammalian stem cells supports a regulatory role for retrotransposons in pluripotency maintenance. Nat Genet 46, 558-66 (2014).
47. Lu, X. et al. The retrovirus HERVH is a long noncoding RNA required for human embryonic stem cell identity. Nat Struct Mol Biol 21, 423-5 (2014).
48. Hall, L.L. et al. Stable C0T-1 repeat RNA is abundant and is associated with euchromatic interphase chromosomes. Cell 156, 907-19 (2014).
49. Jachowicz, J.W. et al. LINE-1 activation after fertilization regulates global chromatin accessibility in the early mouse embryo. Nat Genet 49, 1502-1510 (2017).
50. Fadloun, A. et al. Chromatin signatures and retrotransposon profiling in mouse embryos reveal regulation of LINE-1 by RNA. Nat StructMol Biol 20, 332-8 (2013).
51. Percharde, M. et al. A LINE1-Nucleolin Partnership Regulates Early Development and ESC Identity. Cell 174, 391-405 e19 (2018).
52. Quezada, S.A. & Peggs, K.S. Tumor-reactive CD4+ T cells: plasticity beyond helper and regulatory activities. Immunotherapy 3, 915-7 (2011).
53. Catalano, I., Grassi, E., Bertotti, A. & Trusolino, L. Immunogenomics of Colorectal Tumors: Facts and Hypotheses on an Evolving Saga. Trends Cancer 5, 779-788 (2019).
54. Jerby-Arnon, L. et al. A Cancer Cell Program Promotes T Cell Exclusion and Resistance to Checkpoint Blockade. Cell 175, 984-997 e24 (2018).
55. Jamal-Hanjani, M., Thanopoulou, E., Peggs, K.S., Quezada, S.A. & Swanton, C. Tumour heterogeneity and immune-modulation. Curr Opin Pharmacol 13, 497-503 (2013).
56. Munn, D.H. & Bronte, V. Immune suppressive mechanisms in the tumor microenvironment. Curr Opin Immunol 39, 1-6 (2016).
57. Yang, Y. Cancer immunotherapy: harnessing the immune system to battle cancer. J Clin Invest 125, 3335-7 (2015).
58. Galon, J. & Bruni, D. Approaches to treat immune hot, altered and cold tumours with combination immunotherapies. Nat Rev Drug Discov 18, 197-218 (2019).
59. Guo, X. et al. Global characterization of T cells in non-small-cell lung cancer by single-cell sequencing. Nat Med 24, 978-985 (2018).
60. Lavin, Y. et al. Innate Immune Landscape in Early Lung Adenocarcinoma by Paired Single-Cell Analyses. Cell 169, 750-765 e17 (2017).
61. Lei Zhang, X.Y., Liangtao Zheng, Yuanyuan Zhang, Yansen Li, Qiao Fang, Ranran Gao, Boxi Kang, Qiming Zhang, Julie Y. Huang, Hiroyasu Konno, Xinyi Guo, Yingjiang Ye, Songyuan Gao, Shan Wang, Xueda Hu, Xianwen Ren, Zhanlong Shen, Wenjun Ouyang & Zemin Zhang. Lineage tracking reveals dynamic relationships of t cells in colorectal cancer. Nature (2018).
62. Savas, P. et al. Single-cell profiling of breast cancer T cells reveals a tissue-resident memory subset associated with improved prognosis. Nat Med 24, 986-993 (2018).
63. Bray, F. et al. Global cancer statistics 2018: GLOBOCAN estimates of incidence and mortality worldwide for 36 cancers in 185 countries. CA Cancer J Clin 68, 394-424 (2018).
64. Travis, W.D. Pathology of lung cancer. Clin Chest Med 23, 65-81, viii (2002).
65. Siegel, R.L., Miller, K.D. & Jemal, A. Cancer statistics, 2018. CA Cancer J Clin 68, 7-30 (2018).
66. Ostertag, E.M. & Kazazian, H.H., Jr. Biology of mammalian L1 retrotransposons. Annu Rev Genet 35, 501-38 (2001).
67. Han, J.S., Szak, S.T. & Boeke, J.D. Transcriptional disruption by the L1 retrotransposon and implications for mammalian transcriptomes. Nature 429, 268-74 (2004).
68. Liu, N. et al. Selective silencing of euchromatic L1s revealed by genome-wide screens for L1 regulators. Nature 553, 228-232 (2018).
69. Ricciardi, S. et al. The Translational Machinery of Human CD4(+) T Cells Is Poised for Activation and Controls the Switch from Quiescence to Metabolic Remodeling. Cell Metab 28, 961 (2018).
70. Werner, M.S. & Ruthenburg, A.J. Nuclear Fractionation Reveals Thousands of Chromatin-Tethered Noncoding RNAs Adjacent to Active Genes. Cell Rep 12, 1089-98 (2015).
71. Bushnell, B. BBTools Software Package. (2017).
72. Dobin, A. et al. STAR: ultrafast universal RNA-seq aligner. Bioinformatics 29, 15-21 (2013).
73. Li, H. et al. The Sequence Alignment/Map format and SAMtools. Bioinformatics 25, 2078-9 (2009).
74. Grabherr, M.G. et al. Full-length transcriptome assembly from RNA-Seq data without a reference genome. Nat Biotechnol 29, 644-52 (2011).
75. Haas, B.J. et al. Improving the Arabidopsis genome annotation using maximal transcript alignment assemblies. Nucleic Acids Res 31, 5654-66 (2003).
76. Pertea, M. et al. StringTie enables improved reconstruction of a transcriptome from RNA-seq reads. Nat Biotechnol 33, 290-5 (2015).
77. Quinlan, A.R. & Hall, I.M. BEDTools: a flexible suite of utilities for comparing genomic features. Bioinformatics 26, 841-2 (2010).
78. Cabili, M.N. et al. Integrative annotation of human large intergenic noncoding RNAs reveals global properties and specific subclasses. Genes Dev 25, 1915-27 (2011).
79. Hon, C.C. et al. An atlas of human long non-coding RNAs with accurate 5' ends. Nature 543, 199-204 (2017).
80. Panzeri, I., Rossetti, G., Abrignani, S. & Pagani, M. Long Intergenic Non-Coding RNAs: Novel Drivers of Human Lymphocyte Differentiation. Front Immunol 6, 175 (2015).
81. Ranzani, V. et al. The long intergenic noncoding RNA landscape of human lymphocytes highlights the regulation of T cell differentiation by linc-MAF-4. Nat 16, 318-325 (2015).

## Claims

1. A suppressor or inhibitor of (long interspersed element 1) LINE1 (L1) expression for medical use.

2. A suppressor or inhibitor of LINE1 expression for use in the treatment and/or prevention of primary or secondary immunodeficiency, or of pathologies that display an immunosuppressed phenotype, preferably of cancers and/or metastasis, more preferably of lung cancer, even more preferably non-small cells lung carcinoma (NSCLC), or colorectal cancer (CRC), or of viral diseases such as immunodeficiencies due to Human Immunodeficiency Virus (HIV) or Lymphocytic choriomeningitis virus (LCMV).

3. The suppressor or inhibitor for use according to claim 1 or 2, wherein the suppressor or inhibitor is at least one molecule selected from the group consisting of:
a) an antibody or a fragment thereof;
b) a polypeptide;
c) a small molecule;
d) a polynucleotide coding for said antibody or polypeptide or a functional derivative thereof;
e) a polynucleotide, such as antisense construct, antisense oligonucleotide, RNA interference construct or siRNA,
f) a vector comprising or expressing the polynucleotide as defined in d) or e);
g) a CRISPR/Cas9 component, e.g. a sgRNA;
h) a host cell genetically engineered expressing said polypeptide or antibody or comprising the polynucleotide as defined in d) or e) or at least one component of g).

4. The suppressor or inhibitor for use according to claim 3, wherein the polynucleotide is an isolated inhibitory nucleic acid targeting LINE1, preferably wherein
the inhibitory nucleic acid comprises a sequence of nucleotides that are identical or complementary to 10 to 50 consecutive nucleotides of SEQ ID NO: 1, and/or
said inhibitory nucleic acid is at least one RNA inhibitor, preferably selected from the group consisting of: antisense oligo (ASO), gapmer, mixmer, shRNA, siRNA, stRNA, snRNA, more preferably said inhibitory nucleic acid is modified, such as 2'-deoxy-2'-fluoro-β-D-arabinonucleid acid (FANA) ASO, and/or comprises one or more modified bonds or bases.

5. The suppressor or inhibitor for use according to claim 4, wherein said ASO is designed on a consensus sequence of L1, especially L1M subfamilies and ORF2-3'UTR region.

6. The suppressor or inhibitor for use according to claim 4 or 5 wherein the ASO comprises a sequence capable of hybridizing or complementary to the ORF2-3'UTR region of L1, preferably of specific L1 subfamilies, such as L1M subfamilies.

7. The suppressor or inhibitor for use according to claim 4, 5 or 6 wherein the ASO comprises a nucleic acid sequence that targets one of the following sequences or the corresponding RNA sequence:
LINE1-a GCACTAAATGCCTACAAGAGA (SEQ ID NO:2)
LINE1-b GATAGACCGCTAGCAAGACTA (SEQ ID NO:3)
LINE1-c GAAGTTGAATCTCTGAATAGA (SEQ ID NO:4)
LINE1-d GGACCTCTTCAAGGAGAACTA (SEQ ID NO:5)
LINE1-e GGAGAGGATGCGGAGAAATAG (SEQ ID NO:6)

8. The suppressor or inhibitor for use according to any one of claims 1-7, being used in T cells, preferably CD4+ T naive cells or a CD8+ T cell, Tumor infiltrating Lymphocytes TILs both CD4+ and CD8+, B cells, Natural Killer cells or Tumor cells.

9. The suppressor or inhibitor for use according to any one of claims 1-8 in combination with an immunotherapy and/or with a radiotherapy and/or chemotherapeutic agent and/or with targeted therapies which promote raising of new antigens and immunity response and/or with immunity system adjuvants, preferably said immunotherapy comprises administration of an immune checkpoint inhibitor and/or chimeric antigen receptor (CAR)-expressing immune effector cells, preferably the immune checkpoint inhibitor is an or comprises one or more anti-CD137 antibodies; anti-PD-1 (programmed cell death 1) antibodies; anti-PDLl (programmed cell death ligand 1) antibodies; anti-PDL2 antibodies; or anti-CTLA-4 antibodies.

10. The suppressor or inhibitor for use according to any one of claims 1-9, being used in Adoptive cell transfer (ACT), cell therapy treatment, mismatched bone marrow transplantation, mismatched NK cell infusion or cytokine-induced killer (CIK) cell infusion, or wherein said suppressor or inhibitor is injected in the tumour site, e.g. in intestine tumour, melanoma, or delivered by nanoparticles specifically to the site of interest.

11. A pharmaceutical composition comprising the suppressor or inhibitor as defined in any one of claims 1-10 and at least one pharmaceutically acceptable carrier, and optionally further comprising a therapeutic agent.

12. A method to modulate the commitment of naive CD4+ T naive cells towards any effector lineage and to modulate the effector response in dysfunctional T cells comprising the step of inhibiting LINE1 expression in said cells, wherein the step of inhibiting LINE1 expression in said cells is performed by means of at least one suppressor or inhibitor as defined in any one of claims 1-10.

13. An isolated human T cell, B cell, NK cell or Tumor cell, wherein said cell is stably or transiently affected in the expression of LINE1, preferably said cell is a CD4+ T naive cell or a CD8+ T cell, or a dysfunctional T cell, e.g. a TIL.

14. A composition comprising at least one cell or combinations thereof as defined in claim 13, said composition preferably further comprising at least one physiologically acceptable carrier.

15. The cell according to claim 13, or the composition according to claim 14 for use as a medicament, preferably for use in the treatment and/or prevention of primary or secondary immunodeficiency, or of pathologies that display an immunosuppressed phenotype, preferably of cancers and/or metastasis, more preferably of lung cancer, even more preferably non-small cells lung carcinoma (NSCLC), or colorectal cancer (CRC), or of viral diseases such as immunodeficiencies due to HIV, Lymphocytic choriomeningitis virus (LCMV).
preferably said cell or composition being used in Adoptive cell transfer (ACT), cell therapy treatment, mismatched bone marrow transplantation, mismatched NK cell infusion or cytokine-induced killer (CIK) cell infusion, or wherein said cell or composition is injected in the tumour site, e.g. in intestine tumour, melanoma, or delivered by nanoparticles specifically to the site of interest.
